# EUROPEAN PATENT SPECIFICATION

(11) **EP 1 212 428 B1**
(45) Date of publication and mention of the grant of the patent: **01.12.2004**
(21) Application number: 00913305.9
(22) Date of filing: 02.02.2000
(51) Int. Cl.: C12N 15/38, C12N 15/86, C12N 7/04, C07K 14/035, A61K 48/00

(54) **CELL-SPECIFIC AND/OR TUMOR-SPECIFIC PROMOTER RETARGETING OF HERPES GAMMA 34.5 GENE EXPRESSION**
ZELL- UND/ODER TUMOR-SPEZIFISCHE PROMOTOR-ABHÄNGIGE ÄNDERUNG DER GEZIELTEN EXPRESSION DES HERPES GAMMA 34.5 GENS
RECIBLAGE DU PROMOTEUR SPECIFIQUE DE TUMEUR ET/OU DE CELLULE DANS L'EXPRESSION DU GENE GAMMA 34.5 DE L'HERPES

(30) Priority: 31.08.1999 US 151621 P
(43) Date of publication of application: 12.06.2002
(73) Proprietor: THE GENERAL HOSPITAL CORPORATION, Boston, MA 02114 (US)
(72) Inventor: CHIOCCA, E., Antonio, Wakefield, MA 01880 (US); CHUNG, Richard, Y., Boston, MA 02114 (US)
(74) Representative: Chapman, Paul William
(86) International application number: PCT/US2000/002409
(87) International publication number: WO 2001/016331

(56) References cited:
- WO-A-96/00007
- WO-A-96/03997
- WO-A-97/04804
- WO-A-98/42195
- C.M. KRAMM ET AL.: "Therapeutic and safety of a second-generation replication-conditional HSV1 vector for brain tumor gene therapy" HUMAN GENE THERAPY, vol. 8, no. 17, 20 November 1997 (1997-11-20), pages 2057-2068, XP000929872 MARY ANN LIEBERT, INC. PUBLISHERS, NEW YORK, US cited in the application
- CHAMBERS R ET AL: "Comparison of genetically engineered herpes simplex viruses for the treatment of brain tumors in a scid mouse model of human malignant glioma" PROCEEDINGS OF THE NATIONAL ACADEMY OF SCIENCES OF USA,US,NATIONAL ACADEMY OF SCIENCE. WASHINGTON, vol. 92, no. 5, 28 February 1995 (1995-02-28), pages 1411-1415, XP002090032 ISSN: 0027-8424 cited in the application
- PERNG ET AL: "AN AVIRULENT ICP34.5 DELETION MUTANT OF HERPES SIMPLEX VIRUS TYPE 1 IS CAPABLE OF IN VIVO SPONTANEOUS REACTIVATION" JOURNAL OF VIROLOGY,US,THE AMERICAN SOCIETY FOR MICROBIOLOGY, vol. 69, no. 5, 1 May 1995 (1995-05-01), pages 3033-3041, XP002090031 ISSN: 0022-538X
- LAM E W -F ET AL: "Cell-cycle regulation of human B-myb transcription" GENE,NL,ELSEVIER BIOMEDICAL PRESS. AMSTERDAM, vol. 160, no. 2, 28 July 1995 (1995-07-28), pages 277-281, XP004042159 ISSN: 0378-1119
- CHUNG R Y ET AL: "B-myb promoter retargeting of herpes simplex virus gamma34.5 gene-mediated virulence toward tumor and cycling cells." JOURNAL OF VIROLOGY, (1999 SEP) 73 (9) 7556-64. JOURNAL CODE: KCV. , XP002144570
- NAKAMURA HIDEO ET AL: "Oncolysis of colon carcinoma liver metastasis in genetically-engineered HSV-1 vector Myb34.5." PROCEEDINGS OF THE AMERICAN ASSOCIATION FOR CANCER RESEARCH ANNUAL, no. 41, March 2000 (2000-03), page 466 XP002144571 91st Annual Meeting of the American Association for Cancer Research.;San Francisco, California, USA; April 01-05, 2000, March, 2000 ISSN: 0197-016X

## Description

At least part of the work performed during development of this invention utilized a grant from the National Cancer Institute, Grant No. CA6924602. The U.S. Government has certain rights in this invention.

### Background of the Invention

### Field of the Invention

The present invention relates to a herpes viral mutant capable of selectively targeting tumor cells and/or other specific cell populations. More particularly, the present invention relates to the use of cell-specific and/or tumor-specific promoters to retarget mutant herpes viral vectors toward tumors and specific cell types. The cell-specific and/or tumor-specific promoter is used to drive expression of the herpes gamma (γ) 34.5 gene, whose gene product is responsible for producing large quantities of progeny virus in infected cells.

Herpes vectors without the γ34.5 gene do not replicate well, which is desirable for clinical use. However, the absence ofthe γ34.5 gene, also diminishes the ability of the virus to kill tumors or any other infected tissue. The present invention allows for the production of high amounts of herpes virus in cells that can use the cell- and/or tumor-specific promoter. Cells that cannot turn on the promoter, however, do not support viral replication, thus saving them and their neighboring cells from active and noxious viral infection and replication, thus, redirecting herpes' virulence towards desired target cells.

### Related Art

### A. Conventional Cancer Therapies

Neoplasia is a process that occurs in cancer, by which the normal controlling mechanisms that regulate cell growth and differentiation are impaired, resulting in progressive growth. This impairment of control mechanisms allows a tumor to enlarge and occupy spaces in vital areas of the body. If the tumor invades surrounding tissue and is transported to distant sites (metastases) it will likely result in death of the individual.

In 1999, in the United States alone, approximately 563,100 people, or about 1,500 people per day, are expected to die of cancer. (Landis, *et al*., "Cancer Statistics, 1999," *CA Canc*. *J*. *Clin*. *49*:8-31 (1999)). Moreover, cancer is a leading cause of death among children aged 1 to 14 years, second only to accidents. *Id*. Thus, clearly there is a need for the development of new cancer therapies.

### 1. Common Limitations of Conventional Therapies

The desired goal of cancer therapy is to kill cancer cells preferentially, without having a deleterious effect on normal cells. Several methods have been used in an attempt to reach this goal, including surgery, radiation therapy, and chemotherapy.

Surgery was the first cancer treatment available, and still plays a major role in diagnosis, staging, and treatment of cancer, and may be the primary treatment for early cancers (*see,* Slapak, C.A. and Kufe, D.W., "Principles of Cancer Therapy," in *Harrison's Principles of Internal Medicine*, Fauci, A.S. *et al*., eds., 14th Ed., McGraw-Hill Cos., Inc., New York, 1998, at 524). However, although surgery may be an effective way to cure tumors confined to a particular site, these tumors may not be curable by resection due to micrometastatic disease outside the tumor field. *Id*. Any cancer showing a level of metastasis effectively cannot be cured through surgery alone. *Id*.

Radiation therapy is another local (nonsystemic) form of treatment used for the control of localized cancers. *Id*. at 525. Many normal cells have a higher capacity for intercellular repair than neoplastic cells, rendering them less sensitive to radiation damage. Radiation therapy relies on this difference between neoplastic and normal cells in susceptibility to damage by radiation, and the ability of normal organs to continue to function well if they are only segmentally damaged. *Id*. Thus, the success of radiation therapy depends upon the sensitivity of tissue surrounding the tumor to radiation therapy. *Id*. Radiation therapy is associated with side effects that depend in part upon the site of administration, and include fatigue, local skin reactions, nausea and vomiting. *Id*. at 526. In addition, radiation therapy is mutagenic, carcinogenic and teratogenic, and may place the patient at risk of developing secondary tumors. *Id*.

Other types of local therapy have been explored, including local hyperthermia (Salcman, M., *et al*., *J*. *Neuro-Oncol*. *1*:225-236 (1983)), photoradiation therapy (Cheng, M.K., *et al*., *Surg. Neurol. 25*:423-435 (1986)), and interstitial radiation (Gutin, P.H., *et al*., *J*. *Neurosurgery 67*:864-873 (1987)). Unfortunately, these therapies have been met with only moderate success.

Local treatments, such as radiation therapy and surgery, offer a way of reducing the tumor mass in regions of the body that are accessible through surgical techniques or high doses of radiation therapy. However, more effective local therapies with fewer side effects are needed. Moreover, these treatments are not applicable to the destruction of widely disseminated or circulating tumor cells eventually found in most cancer patients. To combat the spread of tumor cells, systemic therapies are used.

One such systemic treatment is chemotherapy. Chemotherapy is the main treatment for disseminated, malignant cancers (Slapak, C.A. and Kufe, D.W., "Principles of Cancer Therapy," in *Harrison's Principles of Internal Medicine*, Fauci, A.S. *et al*., eds., 14th Ed., McGraw-Hill Cos., Inc., New York, 1998,527). However, chemotherapeutic agents are limited in their effectiveness for treating many cancer types, including many common solid tumors. *Id*. This failure is in part due to the intrinsic or acquired drug resistance of many tumor cells. *Id*. at 533. Another drawback to the use of chemotherapeutic agents is their severe side effects. *Id*. at 532. These include bone marrow suppression, nausea, vomiting, hair loss, and ulcerations in the mouth. *Id*. Clearly, new approaches are needed to enhance the efficiency with which a chemotherapeutic agent can kill malignant tumor cells, while at the same time avoiding systemic toxicity.

### 2. Challenges Presented by Central Nervous System Tumors

Another problem in cancer treatment is that certain types of cancer, e.g., gliomas, which are the most common primary malignancy arising in the human brain, defy the current modalities of treatment. Despite surgery, chemotherapy, and radiation therapy, glioblastoma multiforme, the most common of the gliomas, is almost universally fatal (Schoenberg, in *Oncology of the Nervous System*, M. D. Walker, ed., Boston, Mass., Martinus Nijhoff (1983); Levin *et al*., Chapter 46 in *Cancer: Principles and Practice of Oncology*, vol. 2, 3rd ed., De Vita *et al*., eds., Lippincott Press, Philadelphia (1989), pages 1557-1611).

Gliomas represent nearly 40% of all primary brain tumors, with glioblastoma multiforme constituting the most malignant form (Schoenberg, "The Epidemiology of Nervous System Tumors," in *Oncology of the Nervous System*, Walker, ed., Cold Spring Harbor Laboratory, Cold Spring Harbor, N.Y. (1983)). The five year survival rate for persons with this high grade type of astrocytoma is less than 5 percent, given the current treatment modalities (surgery, radiation therapy and/or chemotherapy) (Mahaley *et al*., *Neurosurgery 71*: 826-836 (1989); Schoenberg, in *Oncology of the Nervous System*, Walker, ed., Cold Spring Harbor Laboratory, Cold Spring Harbor, N.Y. (1983); Kim *et al*., *J*. *Neurosurg. 74:*27-37 (1991), Daumas-Duport *et al*., *Cancer 2*:2152-2165 (1988)). After treatment with radiation therapy, glioblastomas usually recur locally. Hochberg, F.H., *et al*., *Neurology 30*:907-911 (1980). Neurologic dysfunction and death in an individual with glioblastoma are due to the local growth of the tumor. Systemic metastases are rare. *Id*. For this reason, regional cancer therapy methods, rather than systemic methods, may be especially suitable for the treatment of glioblastomas.

Moreover, glioblastomas are resistant to many chemotherapeutic agents, perhaps due to the proliferative characteristics of this tumor type. Many chemotherapeutic agents are cell-cycle-active, *i. e.*, cytotoxic primarily to actively cycling cells (Slapak, C.A., and Kufe, D.W., "Principles of Cancer Therapy," in *Harrison's Principles of Internal Medicine*, Fauci, A.S. *et al*., eds., 14th Ed., McGraw-Hill Cos., Inc., New York, 1998, 527). Generally, chemotherapy is most effective for cancers with a small tumor burden where the growth fraction of the tumor is maximal. *Id*. The growth fraction for glioblastoma tumors is only 30%, with the remaining 70% of cells being in G₀, a resting phase (cells in G₀ may die or may re-enter the active cell cycle (Yoshii *et al*., *J*. *Neurosurg. 65*:659-663 (1986)). While the 30% of glioblastoma cells that are actively dividing contribute to the lethal progression of this tumor, the 70% that are quiescent are responsible for the resistance of these tumors to a number of chemotherapeutic agents that target actively proliferating cells.

Unfortunately, regional treatments, such as surgery and radiation therapy have also found limited success in the treatment of glioblastomas (Burger *et al*., *J*. *Neurosurg*. *58*:159-169 (1983); Wowra *et al*., *Acta Neurochir*. *(Wien) 99*:104-108 (1989); Zamorano *et al*., *Acta Neurochir*. *Suppl*. *(Wien) 46*:90-93 (1989)). Surgical treatment for glioblastomas is hampered by the lack of distinct boundaries between the tumor and the surrounding parenchyma, and by the migration of tumor cells in the white matter tracts extending out from the primary site (Burger *et al*., *J*. *Neurosurg*. *58*:159-169 (1983)), which preclude their Complete removal. Radiation therapy, which targets rapidly proliferating cells, is limited by the low growth fraction in glioblastomas, and by the radiation sensitivity of adjacent normal tissue (Wowra *et al*., *Acta Neurochir*. *(Wien) 99*:104-108 (1989); Zamorano *et al*., *Acta Neurochir*. *Suppl*. *(Wien) 46*:90-93 (1989)). Thus, new approaches are especially needed to treat brain tumors.

### B. Non-traditional Approaches to Cancer Therapy Using Viruses

One non-traditional approach to cancer therapy employs mutated viruses to target neoplastic cells. *See,* Chung, R.Y. and Chiocca, E.A., *Surg. Oncol*. *Clin. N. Am.* 7:589-602 (1998)).

Proposed viral cancer therapies include two distinct approaches: (1 ) direct cell killing (oncolysis) by a mutagenized virus (Martuza *et al*., *Science 252*:854-856(1991); Mineta *et al*., *Nature Med 1*:938-943 (1995); Boviatsis *et al*., *Cancer Res*. *54*: 5745-5751 (1994); Kesari, *et al*., *Lab*. *Invest*. *73*: 636-648 (1995); Chambers *et al*., *Proc*. *Natl. Acad. Sci*. *USA 92*: 1411-1415 (1995); Lorence, R.M. *et al*., *J. Natl*. *Cancer*. *Inst*. *86*: 1228-1233 (1994); Bischoff, *et al*., *Science 274*: 373-376 (1996); Rodriguez *et al*., *Cancer Res. 57*: 2559-2563 (1997)); and (2) the use of viral vectors to deliver a transgene whose expression product activates a chemotherapeutic agent (Wei *et al*., *Human Gene Therapy 5*: 969-978 (1994); Chen and Waxman, *Cancer Res*. *55*: 581-589 (1995); Moolten, *Cancer Gene Ther*. *1*: 279-287 (1994); Fakhrai *et al*., *Proc*. *Natl*. *Acad*. *Sci*. *USA 93*: 2909-2914 (1996); Roth *et al*., *Nature Med*. *2*: 985-991 (1996); Moolten, *Cancer Res*. *46*: 5276-5281 (1986); Chen *et al*., *Proc*. *Natl*. *Acad*. *Sci*. *USA 91*: 3054-3057(1994)).

### 1. Viral Oncolysis

The genetic engineering of viruses for use as oncolytic agents has initially focused on the use of replication-incompetent viruses. This strategy was hoped to prevent damage to non-tumor cells by the viruses. A major limitation of this approach was that these replication-incompetent viruses required a helper virus to be able to integrate and/or replicate in a host cell. One example of the viral oncolysis approach, the use of replication-defective retroviruses for treating nervous system tumors, requires the implantation of a producer cell line to spread the virus. These retroviruses are limited in their effectiveness, because each replication-defective retrovirus particle can enter only a single cell and cannot productively infect others thereafter. Therefore, they cannot spread far from the producer cell, and are unable to completely penetrate a deep, multilayered tumor *in vivo* (Markert *et al*., *Neurosurg*. *77*:590 (1992); Ram *et al*., *Nature Medicine 3*:1354-1361 (1997)).

More recently, genetic engineering of oncolytic viruses has focused on the generation of replication-conditional viruses in an attempt to avoid systemic infection, while allowing the virus to spread to other tumor cells. Replication-conditional viruses are designed to preferentially replicate in actively dividing cells, such as tumor cells. Thus, these viruses should target tumor cells for oncolysis, and replicate in these cells so that the virus can spread to other tumor cells.

Some recent strategies for creating replication-conditional viral mutants as anticancer agents have employed mutations in selected adenoviral or herpes simplex virus type 1 (HSV-1) genes to render them replication-conditional (Martuza, R.L., *et al*., *Science 252:*854-856 (1991); Mineta, T., *et al*., *Nature Med 1*:938-943 (1995); Boviatsis, E.J., *et al*., *Cancer Res*. *54:* 5745-5751 (1994); Kesari, S., *et al*., *Lab*. *Invest*. *73*: 636-648 (1995); Chambers, R., *et al*., *Proc*. *Natl*. *Acad*. *Sci*. *USA 92:* 1411-1415 (1995); Lorence, R.M. *et al*., *J Natl*. *Cancer*. *Inst*. *86:* 1228-1233 (1994); Bischoff, J.R., *et al*., *Science 274*: 373-376 (1996); Rodriguez, R., *et al*., *Cancer Res*. *57*: 2559-2563 (1997)). For example, an adenovirus with a deletion in the E1B-55Kd encoding gene has been shown to selectively replicate in p53-defective tumor cells (Bischoff, J.R., *et al*., *supra*).

Two broad types of replication-conditional HSV mutants in a single gene have been studied to date. The first consists of viral mutants with defects in the function of a viral gene needed for nucleic acid metabolism, such as thymidine kinase (Martuza, R.L., *et al*., *Science 252*:854-856 (1991)), ribonucleotide reductase (RR) (Goldstein, D.J. & Weller, S.K., *J*. *Virol*. *62*:196-205 (1988); Boviatsis, E.J., *et al*., *Gene Ther*. *1*:323-331 (1994); Boviatsis, E.J., *et al*., *Cancer Res*. *54*:5745-5751 (1994); Mineta, T., *et al*., *Cancer Res. 54*:3363-3366 (1994)), or uracil-N-glycosylase (Pyles, R.B. and Thompson, R.I., *J*. *Virol*. *68*:4963-4972 (1994)).

The second consists of viral mutants with defects in the function of the γ34.5 gene (Chambers, R., *et al*., *Proc*. *Natl*. *Acad*. *Sci*. *USA 92*:1411-1415 (1995)), which functions as a virulence factor by markedly enhancing the viral burst size of infected cells through suppression of the shutoff of host protein synthesis (Chou, J., *et al*., *Science 250*:1262-1266 (1990); Chou, J. and Roizman, B., *Proc*. *Natl*. *Acad*. *Sci*. *USA 89*:3266-3270 (1992)).

These single mutant strains have certain inherent limitations, including resistance to ganciclovir and acyclovir for TK mutants (Mineta, T., *et al*., *Cancer Res. 54*:3363-3366 (1994)), the risk of reversion to wild-type by a single recombination event with wild-type virus, and reduced oncolytic efficacy for γ34.5 mutants, at least in certain tumor cell lines (Kramm, C.M., *et al*., *Hum. Gene Ther*. *8*:2057-2068 (1997); Mohr. I. & Bluzman, Y., *EMBO J*. *15*:4759-4766 (1996); Toda, M., *et al*., *Hum*. *Gene Ther*. *9*:2177-2185 (1998)).

In an effort to decrease the risk of wild-type recombination, HSV viruses that are multiply mutated have been developed. These include mutants G207 (Mineta, T., *et al*., *Nat*. *Med*. *1*:938-943 (1995); U.S. Patent 5,585,096, issued December 17, 1996 to Martuza *et al*.), and MGH1 (Kramm, C.M., *et al*., *Hum*. *Gene Ther*. *8*:2057-2068 (1997), which possess deletions of both copies of γ34.5 and an insertional mutation of RR.

Another multiply mutated HSV virus is the γ34.5/uracil DNA glycosylase (UNG) mutant strain 3616UB (Pyles, R.B., *et al*., *Hum*. *Gene Ther*. *8*:533-544 (1997)). These double mutant strains demonstrate markedly reduced neurovirulence upon direct intracranial injection, retain sensitivity to ganciclovir, and show relatively selective replication in tumor cells compared to normal tissues. Such double mutant HSV strains retain the defective γ34.5 gene, thus demonstrating little virulence towards normal tissues. Although, they clearly demonstrate oncolytic effects against tumor cells, such effects are less than those observed in mutants with intact γ34.5 genes (Kramm, C.M., *et al*., *Hum. Gene Ther. 8*:2057-2068 (1997); Qureshi, N. and Chiocca, E.A., *unpublished data*). On the other hand, the toxicity exhibited by an intact γ34.5 gene might reduce the potential application of the latter viruses as oncolytic agents.

### 2. Viral Delivery of Anticancer Transgenes

The second approach in viral cancer therapy is the viral delivery of anticancer transgenes, whereby the phenotype of the target tumor cells is genetically altered to increase the tumor's drug sensitivity and responsiveness. This approach involves directly transferring a "chemosensitization" or "suicide" gene encoding a prodrug activation enzyme to malignant cells, in order to confer sensitivity to otherwise innocuous agents (Moolten, F.L., *Cancer Gene Therapy 1*:279-287 (1994); Freeman, S.M., *et al*., *Semin*. *Oncol*. *23*:31-45 (1996); Deonarain, M. P., *et al*., *Gene Therapy 2*: 235-244 (1995)).

Several prodrug activation genes have been studied for application in cancer gene therapy. In one example, herpes simplex virus thymidine kinase (HSV-TK) in combination with the prodrug ganciclovir represents a prototypic prodrug/enzyme activation system known in the art with respect to its potential applications in cancer gene therapy. HSV-TK phosphorylates the prodrug ganciclovir and generates nucleoside analogs that induce DNA chain termination and cell death in actively dividing cells. Tumor cells transduced with HSV-TK acquire sensitivity to ganciclovir, a clinically proven agent originally designed for treatment of viral infections. Moolten, F.L. and Wells, J.M., *J*. *Natl*. *Cancer Inst*. *82*:297-300 (1990); Ezzeddine, Z.D., *et al*., *New Biol*. *3*:608-614 (1991).

In a second example, the bacterial gene cytosine deaminase (CD) is a prodrug/enzyme activation system that has been shown to sensitize tumor cells to the antifungal agent 5-fluorocytosine as a result of its transformation to 5-flurouracil, a known cancer chemotherapeutic agent (Mullen, C.A., *et al*., *Proc*. *Natl*. *Acad*. *Sci*. *USA 89*: 33-37 (1992); Huber, B.E., *et al*., *Cancer Res*. *53*:4619-4626 (1993); Mullen, *C.A., et al*., *Cancer Res. 54*:1503-1506 (1994)).

Recent studies using these drug susceptibility genes have yielded promising results. *See, e*.*g*., Caruso, M., *et al*., *Proc. Natl*. *Acad*. *Sci. USA 90*:7024-7028 (1993); Oldfield,E., *et al*., *Hum. Gene Ther. 4*: 39 (1993); Culver, *K.*, *Clin. Chem 40*: 510 (1994); O'Malley, Jr., B.W., *et al*., *Cancer Res*. *56*:1737-1741 (1996); Rainov, N.G., *et al*., *Cancer Gene Therapy 3*:99-106 (1996).

Several other prodrug-activating enzyme systems have also been investigated (T. A. Connors, *Gene Ther*. *2*:702-709 (1995)). These include the bacterial enzyme carboxypeptidase G2, which does not have a mammalian homolog, and can be used to activate certain synthetic mustard prodrugs by cleavage of a glutamic acid moiety to release an active, cytotoxic mustard metabolite (Marais, R., *et al*., *Cancer Res*. *56*: 4735-4742 (1996)), and *E*.*coli* nitro reductase, which activates the prodrug CB 1954 and related mustard prodrug analogs (Drabek, D., *et al*., *Gene Ther*. *4*:93-100 (1997); Green, N.K., *et al*., *Cancer Gene Ther*. *4*:229-238 (1997)), some of which may be superior to CB 1954 (Friedlos, F. *et al*., *J Med Chem 40*:1270-1275 (1997)). The principle underlying these approaches to prodrug activation gene therapy is that transduction of a tumor cell population with the foreign gene confers upon it a unique prodrug activation capacity, and hence a chemosensitivity which is absent from host cells that do not express the gene.

More recently, a drug activation/gene therapy strategy has been developed based on a cytochrome P450 gene ("CYP" or "P450") in combination with a cancer chemotherapeutic agent that is activated through a P450-catalyzed monoxygenase reaction (Chen, L. and Waxman, D.J., *Cancer Research 55*:581-589 (1995); Wei, M.X., *et al*., *Hum. Gene Ther. 5*:969-978 (1994); U.S. Patent 5,688,773, issued November 18, 1997). Unlike the prodrug activation strategies mentioned above, the P450-based drug activation strategy utilizes a mammalian drug activation gene (rather than a bacterially or virally derived gene), and also utilizes established chemotherapeutic drugs widely used in cancer therapy.

Many anti-cancer drugs are known to be oxygenated by cytochrome P450 enzymes to yield metabolites that are cytotoxic or cytostatic toward tumor cells. These include several commonly used cancer chemotherapeutic drugs, such as cyclophosphamide (CPA), its isomer ifosfamide (IFA), dacarbazine, procarbazine, thio-TEPA, etoposide, 2-aminoanthracene, 4-ipomeanol, and tamoxifen (LeBlanc, G.A. and Waxman, D.J., *Drug Metab*. *Rev*. *20*:395-439 (1989); Ng, S.F. and Waxman D.J., *Intl*. *J*. *Oncology 2*:731-738 (1993); Goeptar, A.R., *et al*., *Cancer Res. 54:*2411-2418 (1994); van Maanen, J.M., *et al*., *Cancer Res*. *47*:4658-4662 (1987); Dehal, S.S., *et al*., *Cancer Res*. *57*:3402-3406 (1997); Rainov, N.G., *et al*., *Human Gene Therapy 9*:1261-1273 (1998)).

In one example of this approach, tumor cells were rendered highly sensitive to CPA or IFA by transduction of CYP2B 1, which encodes a liver P450 enzyme that exhibits a high rate of CPA and IFA activation (Clarke, L. and Waxman, D.J., *Cancer Res. 49*:2344-2350 (1989); Weber, G.F. and Waxman, D.J., *Biochem*. *Pharmacol*. *45*:1685-1694 (1993)). This enhanced chemosensitivity has been demonstrated both *in vitro* and in studies using a subcutaneous rodent solid tumor model and human breast tumor grown in nude mice *in vivo*, and is strikingly effective in spite of the presence of a substantial liver-associated capacity for drug activation in these animals (Chen, L., *et al*., *Cancer Res*. *55*:581-589 (1995); Chen, L., *et al*., *Cancer Res*. *56*:1331-1340 (1996)). This P450-based approach also shows significant utility for gene therapy applications in the treatment of brain tumors (Wei, M.X., *et al*., *Human Gene Ther*. *5*:969-978 (1994); Manome, Y., *et al*., *Gene Therapy 3*:513-520 (1996); Chase, M., *et al*., *Nature Biotechnol*. *16*:444-448 (1998)).

In addition to transgenes comprising prodrug-activating or "suicide" genes, other types of anticancer transgenes have also been studied, including, cytokine genes (to enhance immune defense against the tumor) (Blankenstein, T., *et al*., *J*. *Exp*. *Med*. *173*:1047-1052 (1991); Colombo, M.P., *et al*., *Cancer Metastasis Rev*. *16*:421-432 (1997); Colombo, M.P., *et al*., *Immunol*. *Today 15*:48-51 (1994)) as well as other tumor toxic genes, such as diptheria toxin (Coll-Fresno, P.M., *et al*., *Oncogene 14*:243-247 (1997)), pseudomonas toxin, anti-angiogenesis genes, tumor vaccination genes, tumor suppressor genes, radiosensitivity genes, antisense RNA, and ribozymes (Zaia, J.A., *et al*., *Ann*. *N*.*Y*. *Acad*. *Sci*. *660*:95-106 (1992)).

While both the virus-based and the gene-based approaches have provided evidence of significant therapeutic effects in animal models of tumors, each method suffers from inherent limitations. Although the viral-based approach theoretically provides the potential for extensive replication of the virus with spread in the tumor mass, its effects are limited by the efficiency of viral infection; the requirement of a helper virus or producer cell line for some viral vectors; tumor cell heterogeneity (Sidranski *et al*., *355*: 846-847 (1992); Bigner *et al*., *J*. *Neuropathol*. *Exp*. *Neurol*. *40*: 201-229 (1981)) for the cellular factor(s) complementing viral mutant growth for other viral vectors; and antiviral immune responses.

In the gene-based approaches tested thus far, the efficiency oftransduction of cells within a tumor mass is limited by the defective nature of the vector. In fact, the majority of positively transduced cells occurs within a few cell layers from the site of vector inoculation (Nilaver *et al*. *Proc*. *Natl*. *Acad*. *Sci*. *USA 21*: 9829-9833 (1995); Muldoon *et al*., *Am. J*. *Pathol*. *147*: 1840-1851 (1995); Ram Z. *et al*., *J Neurosurg*. *82*, 343A (abst.)(1995)). Moreover, even for viral vector systems where a producer cell line is unnecessary, or not killed by the suicide gene/drug combination, viral replication may be inhibited by the drug used. Furthermore, where the suicide-gene/drug combination is TK/GCV, the ability of the drug to kill tumor cells is limited by the stage of the cell cycle of the cells as GCV targets only cells in the process of DNA replication. It is thus unlikely that therapeutic gene delivery by these replication-defective vectors will affect tumor cells distant from the inoculation site, even in instances where the therapeutic gene produces a freely diffusible anticancer agent, such as cytokines or CPA metabolites.

A need therefore continues to exist for a safe and efficacious viral mutant that would provide a means to achieve selective virulence for tumors or other targeted cell populations, while retaining lack of toxicity for normal tissues.

### Summary of the Invention

Accordingly, the present invention overcomes the disadvantages of the prior art by providing a herpes viral mutant that can selectively target neoplastic cells for viral oncolysis by the transcriptional retargeting of γ34.5's action. The herpes viral mutant of the invention can also target other cell populations as well.

In a specific, but non-limiting, example, the inventors reintroduced the γ34.5 gene into a RR/γ34.5 double mutant strain (MGH1) under the transcriptional control of the cell-cycle regulated, cellular B-*myb* promoter. They demonstrated that this novel oncolytic virus (called "Myb34.5") remained as oncolytic as a single RR mutant virus that possessed a wild-type γ34.5 gene, yet retained a favorable toxicity profile similar to that of a γ34.5-deletion mutant. These findings thus show that the transcriptional retargeting of a viral gene responsible for preventing the shutoff of protein synthesis of infected cells provides an avenue for achieving selective oncolysis. Thus, the transcriptional retargeting of γ34.5's expression provides a means to achieve selective virulence for tumors, or other targeted cell populations.

In one embodiment of the invention, the herpes viral mutant comprises a deletion or inactivating mutation in both copies of the gene encoding γ34.5, wherein at least one copy of the γ34.5 gene is reintroduced under the transcriptional control of a cell-specific and/or tumor-specific promoter.

Of course, there may be more than one specific endogenous deletion or inactivating mutation of a herpes viral gene, in addition to the γ34.5 gene. These include deletions in the gene that encodes ribonucleotide reductase (RR), or more particularly the large subunit ofRR. Alternatively, the gene encoding RR encodes the small subunit (UL40). Any other herpes viral genes may also be deleted, such as, *e.g.*, thymidine kinase (TK), uracil DNA glycosylase (UNG), or dUTPase. These viral genes are preferred as the mammalian homologues of these genes are often up-regulated in cells with elevated levels of E2F, such as neoplastic cells, and thus can complement the deleted viral enzyme, thereby promoting selective replication in those cells.

In another embodiment, the herpes mutant ofthe invention is also capable of delivering a transgene whose product could be cytotoxic to tumor cells. For example, the transgene could encode a product capable of activating or enhancing a chemotherapeutic agent (*e.g.*, a suicide gene, such as HSV-TK, CD, or cytochrome P450). Alternatively, the transgene can be a cytokine gene to enhance tumor immunogenicity (*e.g*, tumor necrosis factor alpha (TNF-α), interleukins (IL-2, IL-4), interferon-γ, granulocyte-macrophage colony stimulating factor (GM-CSF)), a tumor suppressor gene, or any other tumoricidal gene known to those skilled in the art, such as diptheria toxin, pseudomonas toxin, anti-angiogenesis genes, tumor vaccination genes, radiosensitivity genes, antisense RNA, or ribozymes. Thus, in this embodiment, the herpes mutant further comprises a transgene encoding a gene product capable of converting a chemotherapeutic agent to its cytotoxic form, a cytokine gene, or any other tumoricidal transgene. The transgene can be inserted in the original γ34.5 deletion or anywhere in the UL40 locus.

The invention provides a preferred embodiment of the foregoing herpes viral mutant where the transgene encodes a suicide gene that activates a chemotherapeutic agent. A particularly preferred example of such a suicide gene is mammalian cytochrome P450. More particularly, this cytochrome P450 may be P450 2B1, or alternatively P450 2B6, P450 2A6, P450 2C6, P450 2C8, P450 2C9, P450 2C11, or P450 3A4. P450 2B1 is particularly preferred. If such a suicide gene is present in the foregoing viral mutant, then the chemotherapeutic agent that would be activated is a member ofthe oxazosphorine class. Particularly, the agent would be cyclophosphamide, ifosfamide, N-methyl cyclophosphamide, methylchloropropylnitrosourea, polymeric cyclophosphamide, polymeric ifosfamide, polymeric N-methyl cyclophosphamide, or polymeric methylchloropropylnitrosourea.

The invention also provides an embodiment of the foregoing herpes viral mutants, wherein the herpes mutant is a herpes simplex virus, and more particularly, wherein the mutant is herpes simplex virus (HSV) type 1 or type 2. HSV-1 is particularly preferred.

In a very preferred embodiment of the invention, the herpes viral mutant is derived from HSV-1, and comprises: (a) a deletion or inactivating mutation in both copies of the gene encoding γ34.5; and (b) an insertion of at least one copy of said γ34.5 gene under transcriptional control of a cell-type specific and/or tumor specific promoter, such that said promoter drives expression of the γ34.5 gene.

In addition to γ34.5, deletions or mutations in other herpes viral genes may also be present in the herpes viral mutant of the invention. Deletions in herpes RR, TK, UNG, or dUTPase are exemplary herpes viral genes.

In this embodiment, the cell-specific promoter or tumor-specific promoter can be any one of the well-characterized regulatory elements controlling tumor-type and/or cell-type specific gene expression. For a review, *see,* Miller, N. and Whelan, J., *Hum*. *Gene Ther*. *8*:803-815 (1997); Walther, W. and Stein, U., *J*. *Mol*. *Med*. *74*:379-392 (1996); Schnierle, B.S. and Groner, B., *Gene Therapy 3*:1069-1073 (1996); Lan, K-H., *et al*., *Cancer Res. 57*:4279-4284 (1997); Clary, B.M., *et al*., Cancer Gene Therapy 7:565-574 (1998); Dachs, G.U., *et al*., *Oncol*. *Res*. *9*:313-325 (1997)).

Representative examples of tumor-specific promoters include, *e*.*g*., DF3 (MUC1) (which is overexpressed in the majority of breast cancers)(Abe, M. and Kufe, D., *Proc*. *Natl*. *Acad*. *Sci*. *USA 90*:282-286 (1993); Manome, Y., *et al*., *Gene Ther*. *2*:685, A051 (1995); Chen, L., *et al*., *J Clin*. *Invest*. *96*:2775-2782 (1995)); AFP (which is overexpressed in hepatoma)(Arbuthnot, P., *et al*., *Hepatology 22*:1788-1796 (1995); Ido, A., *et al*., *Cancer Res*. *55*:3105-3109 (1995)); CEA (which is overexpressed in colon and lung cancers)(Thompson, J.A., *et al*., *J Clin*. *Lab*. *Anal*. *5*:344-366 (1991); Osaki, T., *et al*., *Cancer Res*. *54*:5258-5261 (1994)); PSA (which is overexpressed in prostate cancers)(Lundwall, A., *Biochem*. *Biophys*. *Res*. *Commun*. *161*:1151-1156(1989)); tyrosinase (which is overexpressed in melanomas)(Vile, R.G. and Hart, I.R., *Cancer Res*. *53*:962-967 (1993); Vile, R.G. and Hart, I.R., *Ann*. *Oncol 5 (Suppl*. *4)*:S59-S65 (1994); Hart, I.R., *et al*., *Curr*. *Opin*. *Oncol*. *6*:221-225 (1994)); and c-*erb*B2 (which is overexpressed in breast, pancreatic, ovarian, or gastric carcinomas)(Hollywood, D, and Hurst, H., *EMBO J 12*:2369-2375 (1993)).

In a preferred embodiment, the tumor-specific promoter is B-*myb*. In a particularly preferred embodiment of the foregoing herpes viral mutant, the viral mutant is Myb34.5.

Exemplary cell-specific promoters include the following: vascular endothelial growth factor (VEGF) receptor (flk1) promoter expressed in endothelial cells (Kappel *et al*. *Blood* 93: 4282-4292 (1999); insulin promoter expressed in beta cells of the pancreas (Ray *et al*., *J*. *Surg*. *Res*. *84*: 199-203 (1999); gonadotropin-releasing hormone receptor gene expressed in cells of the hypothalamus (Albarracin *et al*., *Endocrinology 140*: 2415-2421 (1999); matrix metalloproteinase 9 promoter, expressed in osteoclasts and keratinocytes (Munant *et al*., *J. Biol*. *Chem*. 274: 5588-5596 (1999); Parathyroid hormone receptor expressed in bone cells (Amizuma *et al*., *J*. *Clin. Invest*. *103*: 373-381 (1999); dopamine beta-hydroxylase promoter expressed in noradrenergic neurons (Yang *et al*., *J*. *Neurochem. 71*: 1813-1826 (1998).

The present invention also provides a method for selectively killing neoplastic cells that overexpresses a known tumor-specific promoter using the herpes viral mutants described above, comprising: infecting said neoplastic cells with said herpes viral mutant, said viral mutant comprising: (a) a deletion or inactivating mutation in a gene encoding γ34.5; and (b) an insertion of at least one copy of said γ34.5 gene under the transcriptional control of said tumor specific promoter, such that said promoter drives expression of said γ34.5 gene; and selectively killing said neoplastic cells.

Representative examples of tumor-specific promoters include: DF3 (MUC1) (which is overexpressed in the majority of breast cancers)(Abe, M. and Kufe, D., *Proc*. *Natl*. *Acad*. *Sci*. *USA 90*:282-286 (1993); Manome, Y., *et al*., *Gene Ther*. *2*:685, A051 (1995); Chen, L., *et al*., *J*. *Clin*. *Invest*. *96*:2775-2782 (1995)); AFP (which is overexpressed in hepatoma)(Arbuthnot, P., *et al*., *Hepatology 22*:1788-1796 (1995); Ido, A., *el al*., *Cancer Res. 55:*3105-3109 (1995)); CEA (which is overexpressed in colon and lung cancers)(Thompson, J.A., *et al*., *J. Clin. Lab*. *Anal. 5*:344-366 (1991); Osaki, T., *et al*., *Cancer Res. 54*:5258-5261 (1994)); PSA (which is overexpressed in prostate cancers)(Lundwall, A., *Biochem. Biophys. Res. Commun*. *161*:1151-1156(1989)); tyrosinase (which is overexpressed in melanomas)(Vile, R.G. and Hart, I.R., *Cancer Res*. *53*:962-967 (1993); Vile, R.G. and Hart, I.R., *Ann*. *Oncol 5 (Suppl*. *4)*:S59-S65 (1994); Hart, I.R., *et al*., *Curr*. *Opin*. *Oncol*. *6*:221-225 (1994)); and *c-erb*B2 (which is overexpressed in breast, pancreatic, ovarian, or gastric carcinomas)(Hollywood, D, and Hurst, H., *EMBO J 12*:2369-2375 (1993)).

Preferably, the tumor-specific promoter is B-*myb*. In a particularly preferred embodiment of this method, the viral mutant is Myb34.5.

In addition to γ34.5, deletions or mutations in other herpes viral genes may also be present in the herpes viral mutant used in the method of the invention. Deletions in herpes RR, TK, UNG, or dUTPase are exemplary herpes viral genes.

In addition, the invention provides the above method for selectively killing neoplastic cells, wherein said herpes viral mutant further comprises a transgene, wherein the transgene is a suicide gene, a cytokine gene, or any tumoricidal gene. If the transgene is a suicide gene, then the method further comprises contacting the neoplastic cells with a chemotherapeutic agent capable of being activated by said suicide gene and selectively killing the neoplastic cells. The preferred suicide gene is cytochrome P450. P450 2B 1 is particularly preferred. Alternatively, the cytochrome P450 encoded is P450 2B6, P450 2A6, P450 2C6, P450 2C8, P450 2C9, P450 2C11, or P450 3A4. In addition, the chemotherapeutic agent is preferably a member of the oxazosphorine class, particularly cyclophosphamide, ifosfamide, N-methyl cyclophosphamide, methylchloropropylnitrosourea, polymeric cyclophosphamide, polymeric ifosfamide, polymeric N-methyl cyclophosphamide, or polymeric methylchloropropylnitrosourea.

Another embodiment of the present invention is a method for selectively eliminating a target cell population that overexpresses a known cell-specific promoter using the herpes viral mutants of the invention, comprising: infecting said target cells with said herpes viral mutant, said viral mutant comprising: (a) a deletion or inactivating mutation in a gene encoding γ34.5; and (b) an insertion of at least one copy of said γ34.5 gene under the transcriptional control of said cell-specific promoter, such that said promoter drives expression of said γ34.5 gene; and selectively eliminating a target cell population.

Exemplary cell-specific promoters include the following: vascular endothelial growth factor (VEGF) receptor (flk1) promoter expressed in endothelial cells (Kappel *et al*. *Blood 93*: 4282-4292 (1999); insulin promoter expressed in beta cells of the pancreas (Ray *et al*., *J*. *Surg*. *Res*. *84*: 199-203 (1999); gonadotropin-releasing hormone receptor gene expressed in cells of the hypothalamus (Albarracin *et al*., *Endocrinology 140*: 2415-2421 (1999); matrix metalloproteinase 9 promoter, expressed in osteoclasts and keratinocytes (Munant *et al*., *J*. *Biol*. *Chem*. *274*: 5588-5596 (1999); Parathyroid hormone receptor expressed in bone cells (Amizuma *et al*., *J*. *Clin*. *Invest*. *103*: 373-381 (1999); dopamine beta-hydroxylase promoter expressed in noradrenergic neurons (Yang *et al*., *J Neurochem*. *71*: 1813-1826 (1998).

In addition to γ34.5, deletions or mutations in other herpes viral genes may also be present in the herpes viral mutant used in the method of the invention. Deletions in herpes RR, TK, UNG, or dUTPase are exemplary herpes viral genes.

Examplary applications of this embodiment include the following:
1) Treatment options to eliminate a noxious cell population: For example, in conditions where there is exuberant neovascularization of blood vessels, such as cerebral Moya-Moya disease, use of the flk receptor promoter to drive gamma 34.5 gene expression would allow for selective elimination of the blood vessels causing this disease. Another example is in conditions where there is extensive bone remodeling and elimination of bone, such as osteoporosis, the use of the matrix metalloproteinase 9 or the parathyroid hormone receptor to drive expression of gamma 34.5 would eliminate bone osteoclasts from further remodeling of bone.
2) To study developmental processes: In order to study the effect of elimination of a cell population on developmental processes, one could use, for example, the dopamine-beta-hydroxylase promoter to eliminate the noradrenergic neurons and then study the effect on animal development.

Another embodiment of the invention is a pharmaceutical composition containing any of the foregoing viral mutants, wherein this composition may also contain one or more pharmaceutically acceptable excipients.

Other objects, features and advantages of the present invention will become apparent from the following detailed description. It should be understood, however, that the detailed description and specific examples, while indicating preferred embodiments of the invention, are given by way of illustration only, since various changes and modifications within the spirit and scope of the invention will become apparent to those skilled in the art from this detailed description.

### Brief Description of the Figures

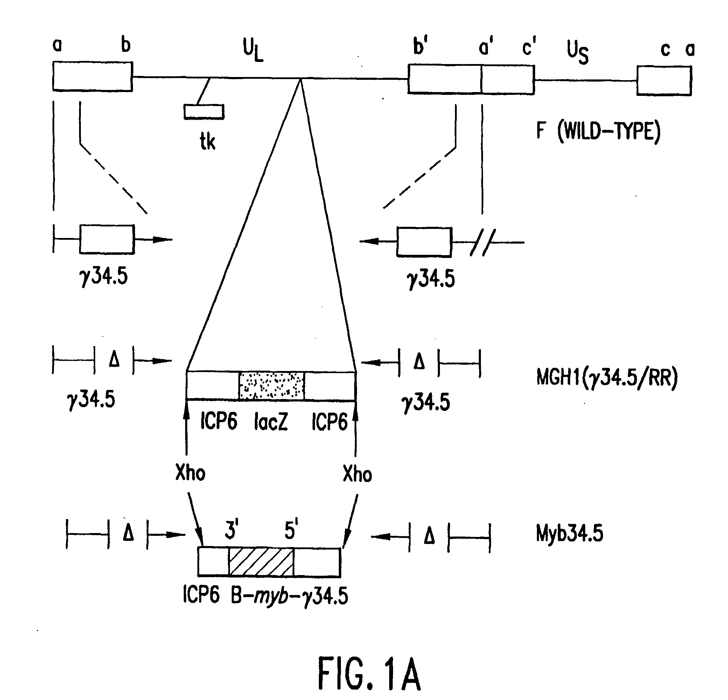

**Figures 1A-1D. Fig. 1A** depicts schematic maps of the HSV strains F (wild-type), MGH1 (double RR(ICP6)/γ34.5 mutant), and Myb34.5. All strains contain the typical HSV genome with two unique segments, UL and US, each flanked by inverted repeat elements, ab and ca, respectively (McGeoch, D.J., *et al*., *J*. *Gen* . *Virol*. *72*:3057-3075 (1991)). Depending on their localization in either unique or repeat segments, the HSV genes are present in one or two copies. The black box indicates the lacZ insertion into a BamHI site within ICP6 (Goldstein, D.J. & Weller, S.K., *J*. *Virol*. *62*:2970-2977 (1988)), while Δ indicates the deletions within γ34.5 in MGH1 and Myb34.5 (Kramm, C.M., *et al*., *Hum*. *Gene Ther*. *8*:2057-2068 (1997)). The hatched bar indicates recombination of the B-*myb* promoter-γ34.5 construct into the ICP6 locus.

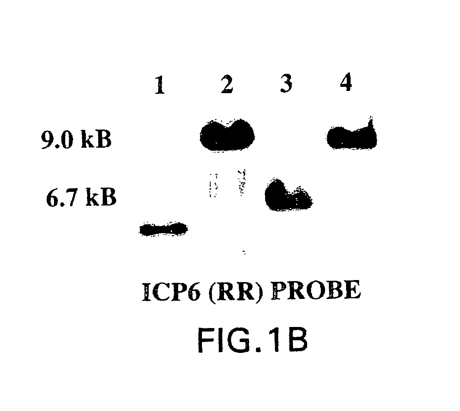

**Fig. 1B** depicts characterization of the HSV mutant Myb34.5 by Southern blot analysis. Hybridization of XhoI digested viral DNA to a full-length probe for ICP6 reveals the expected 9.0 kB fragment sizes for the ICP6 gene with a full length lacZ insertion in MHG1 (lane 2) and MybRevt (lane 4). In Myb 34.5 (lane 3), there is replacement by the B-*myb* promoter/γ34.5 cassette and further deletion of ICP6 to give a 6.7 kB band. DNA from wild-type F strain is in lane 1.

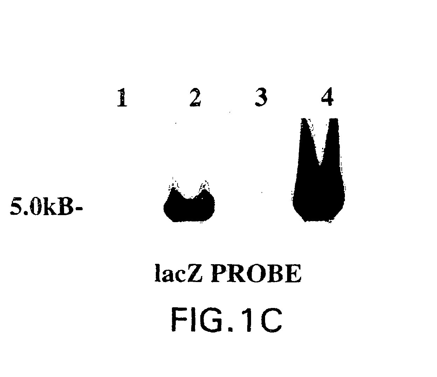

**Fig. 1C** depicts hybridization with a lacZ probe, and reveals fragments with MGH1 (lane 2) and MybRevt (lane 4), with no hybridization to Myb34.5 (lane 3).

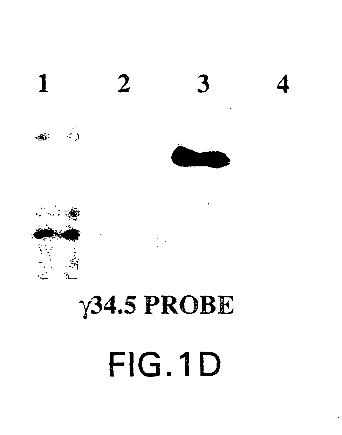

**Fig. 1D** depicts a BstEII-Bbs fragment of γ34.5, internal to the deleted regions of R3616 and MGH1, which reveals a 5.3 kB fragment in BamHI digested Myb34.5 (DNA (lane 3), and several bands in F (lane 1), but fails to hybridize to either MGH1 (lane 2) or MybRevt (lane 4).

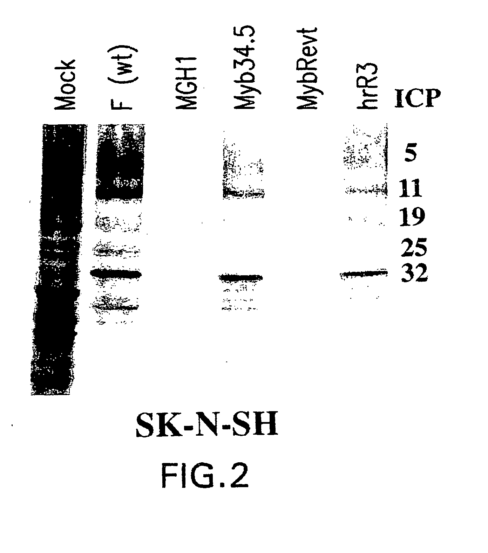

**Figure 2** depicts an autoradiographic image of electrophoretically separated lysates of infected cells demonstrating inhibition of host protein synthesis shutoff by mutant viral strains. Human neuroblastoma cells (SK-N-SH) were plated at 1 x 10⁶ cells/100mm dish. Twenty-four hours later cells were infected at an MOI of 3.0. Fifteen hours following viral infection, cells were briefly washed with methionine-free medium then incubated for 90 minutes with media containing 60 µCi S³⁵-methionine (Toda, M., *et al*., *Hum*. *Gene*. *Ther*. *9*:2177-2185 (1998)). After labeling, cells were harvested, solubilized in a buffer containing SDS, and electrophoresed on 10% acrylamide gels. The gels were then transferred to nitrocellulose and subjected to autoradiography. Infected cell polypeptides were designated according to Morse, L.S., *et al*., *J. Virol*. *26:*389-410 (1978).

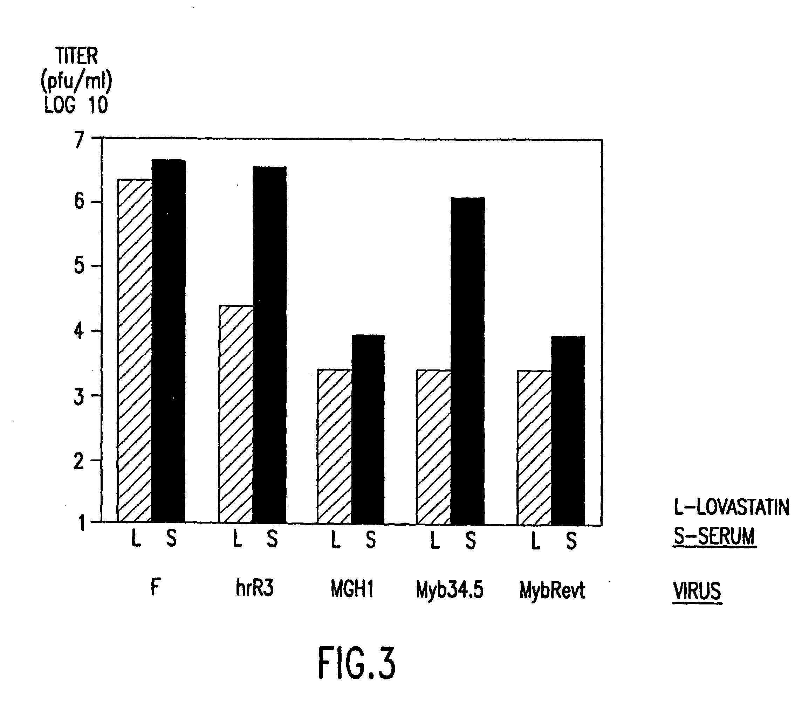

**Figure 3** is a bar graph depicting viral replication in arrested and cycling cells. Human embryo-derived primary fibroblasts (CRL 7706) were plated at 1 x 10⁵ cells/60 mm dish. Forty-eight hours after plating, media was replaced with DMEM containing 20µM Lovastatin for 36 hours (hatched bars). Triplicate plates were counted and infected at a multiplicity of infection (MOI) of 1.0 with various mutant strains (triplicate experiments). Forty-eight hours after infection, cells and supernatants were harvested and virus liberated by freeze-thaw cycles. Parallel experiments were performed with cells allowed to remain in medium containing 10% fetal bovine serum (solid bars). Viral output was determined by plaque assay on Vero cells and is represented as log 10 PFU/1 x 10⁵ input virus (values reflect averages of triplicate experiments). Lovastatin outputs were statistically lower than those obtained with serum for the tested viruses (Student's t-test values: F, p=0.027; hrR3, p=0.001; MGH1, p=0.011; Myb34.5, p=0.001; MybRevt, p=0.003).

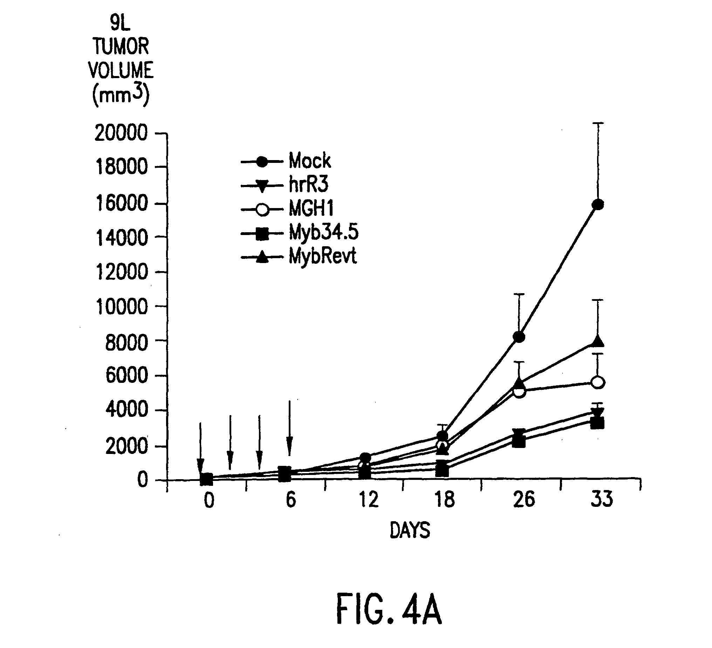

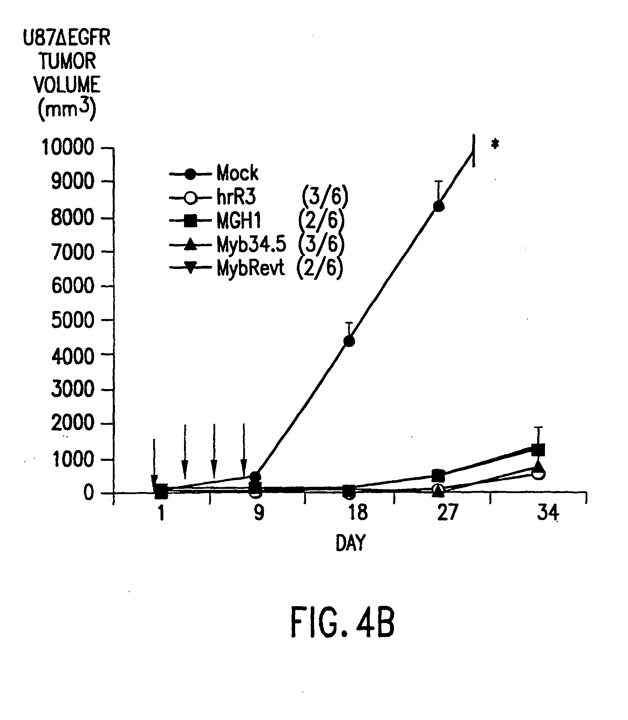

**Figures 4A and 4B** depict *in vivo* growth inhibition by Myb34.5. In similar experiments, rat gliosarcoma 9L (Fig. 4A) and human U87ΔEGFR glioma (Fig. 4B) cells were implanted subcutaneously into the flanks of nude mice. Beginning fourteen (9L) and ten (U87) days later (day 1), vehicle or mutant viral strains were inoculated intratumorally into tumors. Arrows indicate the times of viral injection (days 1, 3, 5, 7), while values are the averages of five mice per group (9L) and six per group (U87ΔEGFR).
In **Fig. 4A**, differences in tumor volumes were significant at the 12, 18, and 33-day time points (p<0.05, one-way repeated measures of variance). In **Fig. 4B,** differences in tumor volumes were significant at the 18, 27, and 34-day time points (p<0.05, one-way repeated measures of variance). For day 34 alone, as shown in Table 4, p<0.005.

### Detailed Description of the Preferred Embodiments

The present invention relates to the selective killing of neoplastic cells by viral mediated oncolysis alone or the combination of viral mediated oncolysis and suicide gene therapy. The invention provides for a herpes viral mutant, a method of selectively killing neoplastic cells using this herpes viral mutant, and a pharmaceutical composition containing the viral mutant. The invention also provides a method for selectively eliminating target cell populations using the herpes viral mutant of the invention.

More specifically, the invention provides for a herpes viral mutant, wherein the mutant is genetically engineered to have (a) a deletion or inactivating mutation in both copies of the gene encoding γ34.5; and (b) an insertion of at least one copy of said γ34.5 gene under the transcriptional control of a tumor-specific or cell-type specific promoter, such that said promoter drives expression of the γ34.5 gene. The herpes mutant may also contain one or more additional deletions or mutations in other herpes viral genes, such as, *e.g.,* RR, TK, UNG, and dUTPase. The herpes viral mutant can also deliver a transgene encoding a product that activates a chemotherapeutic agent, a cytokine gene, or any other tumoricidal gene.

### Design of the Herpes Viral Mutant

The herpes viral mutants of the invention may be derived from several different types of herpes viruses. Herpes viruses that may be used to derive the viral mutants of the invention include herpes simplex virus (HSV), cytomegalovirus, Epstein-Barr virus, varicella zoster virus, and pseudorabies virus.

Herpes simplex viruses are of particular interest. By "herpes simplex virus" is intended any member of the subfamily *herpesviridae alpha* containing a deletion or inactivating mutation as described above. A preferred embodiment of the invention employs HSV-1 or HSV-2 to create the herpes viral mutant, with HSV-1 being the most preferred.

HSV-1 is a human neurotropic virus that is capable of infecting virtually all vertebrate cells. Natural infections follow either a lytic, replicative cycle or establish latency, usually in peripheral ganglia, where the DNA is maintained indefinitely in an episomal state. HSV-1 contains a double-stranded, linear DNA genome, 153 kilobases in length, which has been completely sequenced by McGeoch (McGeoch *et al*., *J Gen*. *Virol*. *69*: 1531 (1988); McGeoch *et al*., *Nucleic Acids Res 14*: 1727 (1986); McGeoch *et al*., *J*. *Mol. Biol*. *181*: 1 (1985); Perry and McGeoch, *J*. *Gen*. *Virol*. *69*: 2831 (1988)). DNA replication and virion assembly occurs in the nucleus of infected cells. Late in infection, concatemeric viral DNA is cleaved into genome length molecules which are packaged into virions. In the CNS, herpes simplex virus spreads transneuronally followed by intraaxonal transport to the nucleus, either retrograde or anterograde, where replication occurs.

### The Herpes Gamma (γ) 34.5 Gene

Published results have demonstrated that at least one function of the herpes γ34.5 gene is to preclude the host cell's response to viral infection, namely the triggering of host protein synthesis shutoff in an apoptotic-like response (Chou, J., *et al*., *Science 250*:1262-1266 (1990); Chou, J. and Roizman, B., *Proc*. *Natl*. *Acad*. *Sci*. *USA 89:*3266-3270 (1992); Chou, J., *et al*., *Proc*. *Natl*. *Acad*. *Sci*. *USA 92*:10516-10520 (1995)). A similar function is widespread among pathogenic viruses (Cosentino, G.P., *et al*., *Proc*. *Natl. Acad*. *Sci. USA 92:*9445-9449 (1995); Gale, M., Jr., *et al*., *Mol. Cell Biol*. *18*:5208-5218 (1998); Katze, M.G., *et al*., *Trends Microbiol*. *3*:75-78 (1995); Sharp, T.V., *et al*., *Nuc*. *Acids Res*. *21*:4483-4490(1993)).

While γ34.5 is nonessential for viral growth in culture in Vero cells, it enables the virus to spread in the central nervous system (CNS) of mice, and maps to a region of the HSV genome previously implicated in CNS replication (Markovitz, N.S., *et al*., *J*. *Virol*. *71*:5560-5569 (1997); Centifanto-Fitzgerald, Y.M., *et al*., *J*. *Esp*. *Med*. *155*:475-489 (1982)). This may be due to the fact that the γ34.5-encoded protein inhibits the double-stranded RNA-dependent kinase (PKR). On exposure to double stranded RNA molecules, as seen commonly with viral infection, PKR phosphorylates the alpha subunit of elongation initiation factor eIF-2, resulting in inhibition of protein synthesis (Chou, J., *et al*., *Science* *250*:1262-1266 (1990); Chou, J. and Roizman, B., *Proc*. *Natl*. *Acad*. *Sci*. *USA 89*:3266-3270 (1992); Chou, J., *et al*., *J*. *Virol*. *68*:8304-8311 (1994)). Infection of cells of neuronal origin with mutants incapable of expressing γ34.5 results in shut-off of cellular protein synthesis, with the resultant limitation of viral production.

In summary, in the presence of γ34.5, HSV will prevent apoptosis, thus allowing for production of progeny viruses. In its absence, the cell dies and the infecting HSV cannot generate progeny viruses. Thus, HSV infection/propagation throughout an organ is eliminated.

The tumor- or cell type-specific promoter/γ34.5 approach of the invention, thus allows for production of virus in cells that can use that promoter, but cells that cannot turn on the promoter will not propagate infection.

The herpes viral mutant of the invention comprises a deletion or inactivating mutation in both copies of the γ34.5 gene, wherein at least one copy of the γ34.5 gene is reintroduced under the control of a cell-specific or tumor-specific promoter.

As used herein, the term "deletion" is intended to mean the elimination of nucleic acids from a gene, such as the γ34.5 gene.

As used herein, the term "inactivating mutation" is intended to broadly mean a mutation or alteration to a gene wherein the expression of that gene is significantly decreased, or wherein the gene product is rendered nonfunctional, or its ability to function is significantly decreased.

The term "gene" encompasses both the regions coding the gene product as well as regulatory regions for that gene, such as a promoter or enhancer, unless otherwise indicated.

Ways to achieve such alterations include: (a) any method to disrupt the expression of the product of the gene or (b) any method to render the expressed gene nonfunctional. Numerous methods to disrupt the expression of a gene are known; including the alterations of the coding region of the gene, or its promoter sequence, by insertions, deletions and/or base changes. (*See*, Roizman, B. and Jenkins, F.J., *Science 229*: 1208-1214 (1985)).

### The Cell-Specific and/or Tumor Specific Promoter

In the herpes viral mutant of the invention, a cell-specific and/or tumor-specific promoter is used to drive expression of y34.5. The promoter can be any one of the well-characterized regulatory elements controlling tumor-type and/or cell-type specific gene expression. For a review, *see,* Miller, N. and Whelan, J., *Hum*. *Gene Ther*. *8*:803-815 (1997); Walther, W. and Stein, U., *J*. *Mol*. *Med*. *74*:379-392 (1996); Schnierle, B.S. and Groner, B., *Gene Therapy 3*:1069-1073 (1996); Lan, K-H., *et al*., *Cancer Res*. *57*:4279-4284 (1997); Clary, B.M., *et al*., Cancer Gene Therapy 7:565-574 (1998); Dachs, G.U., *et al*., *Oncol. Res*. *9*:313-325 (1997)).

By "promoter" is intended to mean the DNA region, usually upstream to the coding sequence of a gene or operon, which binds RNA polymerase and directs the enzyme to the correct transcriptional start site.

By "cell-specific promoter" is intended a promoter that directs expression in particular cell types. One skilled in the art would know that a "tumor-specific" promoter can also be considered a "cell-specific" promoter (*i*.*e*., it is specific for tumor cells). However, for clarity, a "cell-specific promoter", as used herein, is intended to exclude tumor-specific promoters, unless otherwise indicated. Exemplary cell-specific promoters include the following: vascular endothelial growth factor (VEGF) receptor (flk1) promoter expressed in endothelial cells (Kappel *et al*. *Blood 93*: 4282-4292 (1999); insulin promoter expressed in beta cells of the pancreas (Ray *et al*., *J*. *Surg*. *Res*. *84*: 199-203 (1999); gonadotropin-releasing hormone receptor gene expressed in cells of the hypothalamus (Albarracin *et al*., *Endocrinology 140*: 2415-2421 (1999); matrix metalloproteinase 9 promoter, expressed in osteoclasts and keratinocytes (Munant *et al*., *J*. *Biol*. *Chem*. *274*: 5588-5596 (1999); parathyroid hormone receptor expressed in bone cells (Amizuma *et al*., *J*. *Clin*. *Invest*. *103*: 373-381 (1999); and dopamine beta-hydroxylase promoter expressed in noradrenergic neurons (Yang *et al*., *J Neurochem*. *71*: 1813-1826 (1998).

Cell-cycle regulated promoters are also a type of cell-specific promoter. Other cell-specific promoters will be known to those skilled in the art.

Applications of this vector embodiment include the elimination of select noxious cell populations in an organ, as well as animal models to study the elimination of select populations in an organ during development. Exemplary applications of this embodiment include the following:
1) Treatment options to eliminate a noxious cell population: In one example, in conditions where there is exuberant neovascularization of blood vessels, such as cerebral Moya-Moya disease, the use of the flk 1 receptor promoter to drive gamma 34.5 gene expression would allow for selective elimination of the blood vessels causing this disease.
   In another example, in conditions where there is extensive bone remodeling and elimination of bone, such as osteoporosis, the use of the matrix metalloproteinase 9 or the parathyroid hormone receptor to drive expression of gamma 34.5 would eliminate bone osteoclasts from further remodeling of bone.
2) To study the effect of elimination of select populations in an organ during development. For example, in order to study the effect of elimination of a cell population on developmental processes, one could use, for example, the dopamine-beta-hydroxylase promoter to eliminate the noradrenergic neurons and then study the effect on animal development.

By "tumor-specific promoter" is intended a promoter that is induced selectively or expressed at a higher level in the target tumor cell than in a normal cell. The tumor targeting specificity for the herpes viral mutant ofthe invention is achieved by the use of tumor-specific promoters to selectively activate expression ofthe transduced gene in the tumor cell at either the primary tumor site or its metastases (Miller, N. and Whelan, J., *Hum*. *Gene Ther*. *8*:803-815 (1997); Walther, W. and Stein, U., *J*. *Mol*. *Med*. *74*:379-392 (1996); Schnierle, B.S. and Groner, B., *Gene Therapy 3*:1069-1073 (1996); Lan, K-H., *et al*., *Cancer Res. 57*:4279-4284 (1997); Dachs, G.U., *et al*., *Oncol*. *Res*. *9*:313-325 (1997)).

Examples of tumor-specific promoters include those that have been derived from genes that encode tyrosinase (allowing for targeting to melanoma) (Vile, R.G. and Hart, I.R., *Cancer Res*. *53*:962-967 (1993); Vile, R.G. and Hart, I.R., *Ann*. *Oncol 5 (Suppl*. *4)*:S59-S65 (1994); Hart, I.R., *er al*., *Curr*. *Opin*. *Oncol*. *6*:221-225 (1994)); c-*erb*B-2 oncogene (targeting to breast, pancreatic, gastric, and ovarian cancers) (Hollywood, D., and Hurst, H., *EMBO J 12*:2369-2375 (1993)); carcinoembryonic antigen (CEA) (targeting to lung and gastrointestinal malignancies, including colon, pancreatic, and gastric cancer) (Thompson, J.A., *et al*., *J*. *Clin*. *Lab*. *Anal*. *5*:344-366 (1991); Osaki, T., *et al*., *Cancer Res. 54*:5258-5261 (1994)); DF3/MUC 1 (targeting to breast cancer) (Abe, M. and Kufe, D., *Proc*. *Natl*. *Acad*. *Sci*. *USA 90*:282-286 (1993); Manome, Y., *et al*., *Gene Ther. 2*:685, A051 (1995); Chen, L., *et al*., *J Clin*. *Invest*. *96*:2775-2782 (1995)); prostate specific antigen(PSA) (targeting to prostate cancer) (Lundwall, A., *Biochem. Biophys. Res. Commun*. *161*:1151-1156 (1989)); and alpha-fetoprotein (AFP)(targeting to hepatocellular carcinoma) (Arbuthnot, P., *et al*., *Hepatology 22*:1788-1796 (1995); Ido, A., *et al*., *Cancer Res*. *55*:3105-3109 (1995)). The use of synthetic gene regulation systems, which allow for transcriptional control and other forms of regulated expression may also be used (Miller, N. and Whelan, J., *Hum. Gene Ther*. *8*:803-815 (1997); Vile, R.G., *Semin*. *Cancer Biol*. *5*:429-436 (1994); Hwang, J.J., *et al*., *J*. *Virol*. *70*:8138-8141 (1996); Massie, B., *et al*., *J*. *Virol*. *72*:2289-2296 (1998)).

Tumor cells are also known to overexpress particular oncogenes, so that cells with upregulated gene expression can be targeted using promoter elements of such genes. B-*myb*, C-*myb*, c-*myc*, c-*kit*, and the c*-erb*B2 oncogene are some representative examples of these types. The B-*myb* promoter *(see*, Lyon, J., *et* *al*., *Crit*. *Rev*.*Oncogenesis 5*:373-388 (1994) contains a consensus E2F binding site, is strictly regulated in cycling cells, and is in fact repressed in G₀ (Lam, E.W. and Watson, R.J., *EMBO J*. *12*:2705-2713 (1993); Lam, E.W., *et al*., *Gene 160*:277-281 (1995); Bennett, J.D., *et al*., *Oncogene 13*:1073-1082 (1996)). Accordingly, the B-*myb* promoter is a particularly preferred tumor-specific promoter.

Any cancer type having a well-characterized promoter would find use in the invention. Examples of such promoters can be found in Table 1 of Clary, B.M., *et al*., Cancer Gene Therapy 7:565-574 (1998); Table I of Spear, M.A., *Anticancer Research 18*:3223-3232(1998); Table 2 of Walther, W. and Stein, U., *J*.*Mol*.*Med*. *74*:379-392(1996); and Dachs, G.U., *et al*., *Oncol*. *Res*. *9*:313-325 (1997)).

Most if not all of the gene sequences of the tumor-specific promoters described above are available from the GenBank Sequence Database.

### Other Mutations/Deletions in the Construct, in Addition to γ34.5

The viral mutants of the invention may also possess additional mutations in any viral gene(s), but most preferably in a gene required for replication, whose mammalian homologue is up-regulated by elevated levels of E2F.

For example, mammalian ribonucleotide reductase (mRR) is up-regulated during the G₁ phase of the cell cycle and its transcription is regulated by "free" E2F (DeGregori *et al*., *Mol*. *Cell. Biol*. *15*: 4215-4224 (1995); Lukas *et al*., *Mol*. *Cell*. *Biol*. *16*: 1047-1057 (1996); Dynlacht *et al*., *Genes Dev*. *8*: 1772-1786 (1994)). It has been hypothesized that RR⁻ viral mutants selectively replicate in neoplastic cells owing to the presence of the complementing mammalian ribonucleotide reductase (m*RR*))in these cells (Goldstein and Weller, *J*. *Virol*. *62*: 196-205 (1988)).

Elevation in the levels of free E2F causes increased expression of several mammalian genes whose viral homologues are required for replication of the virus. In addition to ribonucleotide reductase (RR), these genes include thymidine kinase (TK), uracyl DNA glycosylase (UNG), and uracyl-triphosphatase enzymes (dUTPase). Viruses containing a mutation in one or more of these genes would replicate selectively in cells with elevated levels of free E2F. Thus, the invention encompasses herpes viral mutants having a mutation in one or more of these genes, in addition to the mutation in γ34.5. In a preferred embodiment of the invention, the mutation is in a ribonucleotide reductase gene.

E2F (including E2F 1, E2F2, E2F3, E2F4, E2F5) appears to be the primary mediator of the cell cycle-regulated transcriptional cascade that involves p 16, cyclin D/ cdk4, and pRB (DeGregori *et al*., *Mol*. *Cell*. *Biol*. *15*: 4215-4224 (1995); Lukas *et al*., *Mol*. *Cell*. *Biol*. *16*: 1047-1057 (1996; Dynlacht *et al*., *Genes Dev. 8:* 1772-1786 (1994)). Thus, defects in a gene involved in this cascade can lead to increased levels of E2F and thereby increased levels of mammalian RR, TK, UNG and dUTPase. For example, cells with defects in the expression of p16, p21 and/or p27 may have increased levels of cyclin D, cyclin D kinase 4 (Cdk4) and/or cyclin D kinase 6 (Cdk6) which may in turn lead to increased phosphorylation ofpRB thereby liberating E2F. In addition, cells with defects in the expression of pRB, p107 and or p130, DP1, DP2, and/or DP3 may also lead to increased liberation of E2F.

The majority of tumors possess an inactivation of a gene encoding a component of this cascade (Ueki *et al*., *Cancer Res. 56:* 150-153 (1996)), thus liberating E2F and allowing for transcription of mammalian RR, TK, UNG, and dUTPase. Moreover, alterations in other tumor suppressor genes or oncogenes may also lead to increased levels of free E2F, and thereby increased levels of mammalian RR, TK, UNG and dUTPase. Therefore, RR⁻, TK⁻, UNG⁻ and dUTPase⁻ viral mutants may effectively target a large percentage of tumor cells, particularly if they possess a defect in the *p16*/ cyclin D/ p*RB* pathway that leads to an increase in "free" E2F.

Furthermore, tumor cells from many different origins (e.g., lung, breast, prostate, brain, liver, pancreas, skin, etc.) possess alterations in the pathways described above leading to elevated levels of RR, TK, UNG and dUTPase, and thus are targets for the viral mutant of the invention. For example, the glioma tumor cell lines (rat 9L, human U87, and human T98 cells) possess inactivating mutations of *p16* (Van Meir *et al*., *Cancer Res*. *54*: 649-652 (1994)), as well as elevated levels of mRR. These cells were thus able to complement the replication of the HSV-1 derived viral mutant rRp450 to levels close to that of the wild-type KOS strain, while neurons with no detectable level of m*RR* (and with a normal *p16* pathway) did not.

By "ribonucleotide reductase gene" is intended a nucleic acid that encodes any subunit or part of the enzyme, ribonucleotide reductase, such that when this nucleic acid is expressed in a cell, this part or subunit is produced, whether functional or nonfunctional. Ribonucleotide reductase (RR) is a key enzyme in the de novo synthesis of DNA precursors, catalyzing the reduction of ribonucleotides to deoxyribonucleotides. HSV-1 encodes its own RR (UL39 and UL40 genes), which is composed of two non- identical subunits (Duita, *J*. *Gen*. *Virol*. *64:* 513 (1983)). The large subunit (140k molecular weight), designated ICP6, is tightly associated with the small subunit (38k molecular weight). Herpes simplex virus RR has been found to be required for efficient viral growth in non-dividing cells but not in many dividing cells (Goldstein and Weller, *J*. *Virol*. *62:* 196 (1988); Goldstein and Weller, *Virol*. *166*: 41 (1988); Jacobson *et al*., *Virol*. *173*: 276 (1989)). Mutations in the small subunit ofRR also lead to loss of RR activity and neuropathogenicity *(Cameron et al*., *J*. *Gen*. *Virol*. *69*: 2607 (1988)), however, particularly preferred are mutations in the large subunit.

The promoter region of ribonucleotide reductase ICP6 has been mapped to the 5' upstream sequences of the ICP6 structural gene (Goldstein and Weller, *J*. *Virol*. *62*: 196 (1988); Sze and Herman, *Virus Res. 26:* 141 (1992)). The transcription start site for the small subunit of RR, namely UL40, falls within the coding region of ICP6 (McLauchlan and Clements, *J*. *Gen*. *Virol*. *64*: 997 (1983); McGeoch *et al*., *J. Gen*. *Virol*. *69*: 1531 (1988)).

Viral mutants derived from HSV-2 based on the viral mutants illustrated herein using the HSV-1 genome are encompassed by the present invention. HSV-2 contains both RR subunits; moreover, HSV-2 ICP10 is analogous to HSV-1 ICP6. Nikas *et al*., *Proteins 1*: 376 (1986); McLaughlan and Clements, *EMBO J*. *2*: 1953 (1983); Swain and Halloway, *J*. *Virol*. *57*: 802 (1986).

One difference between ribonucleotide reductase deficient (RR⁻) and other herpes simplex virus mutants is hypersensitivity to acyclovir and ganciclovir. Because TK⁻ HSV-1 mutants known in the art are resistant to these anti-viral agents, such mutants could be difficult to eliminate in the event of systemic infection or encephalitis. In contrast, in the event of viral encephalitis, TK⁺ viral mutants, such as RR⁻-HSV mutants, are responsive to antiviral therapy.

In addition, RR⁻-HSV mutants are compromised in their ability to produce infections and synthesize viral DNA at 39.5°C *in vitro* (Goldstein and Weller, *Virology 166*:41 (1988)). Therefore, these mutants are attenuated for neurovirulence and less likely to propagate in the event of a fever in the infected host. Such characteristics are important to a therapeutic vector that must be of attenuated neurovirulence and amenable to antiviral therapy in the event of viral encephalitis.

The temperature sensitivity of RR⁻ viral mutants demonstrates another advantage of the viral mutant of the invention. In patients treated with a viral mutant, it is possible that a number of host factors (fever, antiviral immune responses) would inhibit propagation of the viral mutant. In these instances, it would be expected that treatment with a chemotherapeutic agent and activation by the transgene (for those cells infected by the viral mutant) would provide a supplemental anti-cancer treatment.

As used herein, "mutation" refers to any alteration to a gene wherein the expression of that gene is significantly decreased, or wherein the gene product is rendered nonfunctional, or its ability to function is significantly decreased. The term "gene" encompasses both the regions coding the gene product as well as regulatory regions for that gene, such as a promoter or enhancer. Such alterations render the product of the gene non-functional or reduce the expression of the gene such that the viral mutant has the properties of the instant invention. Moreover, the invention encompasses mutants with one or more mutation(s) in one or more gene(s) of interest. Thus, by "a" is intended one or more.

Ways to achieve such alterations include: (a) any method to disrupt the expression of the product of the gene; or (b) any method to render the expressed protein nonfunctional. Numerous methods known to disrupt the expression of a gene are known, including the alterations ofthe coding region of the gene, or its promoter sequence, by insertions, deletions and/or base changes. (*See*, Roizman, B. and Jenkins, F.J., *Science 229*: 1208-1214 (1985)). A deletion is a preferred mutation.

Methods for the construction of engineered viruses and for the genetic manipulation of DNA sequences are known in the art. Generally, these include Ausubel *et al*., Chapter 16 in *Current Protocols in Molecular Biology* (John Wiley and Sons, Inc.); Paoletti *et al*., U.S. Patent 4,603,112 (July 1986). Virological considerations also are reviewed in Coen, in *Virology,* 1990 (2^{nd} ed.) Raven Press, pages 123-150.

The construction of HSV-1 mutants is described, for example, in Martuza *et al*., U.S. Patent 5,585,096 (Dec. 1996); Roizman *et al*., U.S. Patent 5,288,641 (Feb. 1994); Roizman, B. and Jenkins, F.J., *Science 229*:1208-1214 (1985); Johnson *et al*., *J*. *Virol*. *66*:2952 (1992); Gage, P.J., *et al*., *J*. *Virol*. *66*: 5509-5515 (1992); Goldstein and Weller, *J*. *Virol*. *62*:196-205 (1988), Coen, D., Chapter 7, in *Virology*, 1990 (2^{nd} ed.) Raven Press; Breakefield and DeLuca, *The New Biologist 3*:203 (1991); Leib and Olivo, *BioEssays 15*:547 (1993); Glorioso *et al*., *Seminars in Virology 3*:265 (1992); Chou, J. and Roizman, B., *Proc. Natl. Acad*. *Sci. USA*, *89*:3266-3270 (1992); Shih *et al*., in *Vaccines 85*, 1985, Cold Spring Harbor Press, pages 177-180; Kramm, C.M., *et al*., *Hum. Gene Ther. 8*:2057-2068 (1997)); Glorioso, J.C., *et al*., *Annu*. *Rev*. *Microbiol*. *49*:675-710 (1995); Mocarski *et al*., *Cell 22*:243 (1980)).

Genetic alterations of the viral genome can be determined by standard methods such as Southern blot hybridization of restriction endonuclease digested viral DNA, sequencing of mutated regions of viral DNA, detection of new (or lost) restriction endonuclease sites, enzymatic assay for ribonucleotide reductase activity (Huszar, D. and Bacchetti, S., *J*. *Virol*. *37*:580-598 (1981)). For cells lacking the mammalian homologue of the mutated viral gene, *e*.*g*., RR, genetic alteration of the viral genome can be determined by (1) Western blot or ELISA analysis of infected cell proteins with antibodies the viral homologue that has been mutated, *e.g.*, RR, or (2) Northern blot analysis of infected cells for transcription of the viral homologue that has been mutated, *e.g.*, RR (Jacobson, J.G., *et al*., *Virology 173*:276-283 (1989)). A viral mutant that has been mutated in one or more genes can be isolated after mutagenesis or constructed via recombination between the viral genome and genetically-engineered sequences.

By "up-regulated" is intended that expression of the gene(s) encoding the gene product said to be up-regulated is greater than the basal level of expression of this product as found in non-neoplastic cells.

By "level of free E2F is elevated" is meant that the amount of unbound E2F available in a cell is greater than the amount typically found in non-neoplastic cells.

By "selectively killing neoplastic cells" is meant that the herpes viral mutant of the invention primarily targets neoplastic cells, rather than non-neoplastic cells.

By "neoplastic cells" is meant cells whose normal growth control mechanisms are disrupted (typically by accumulated genetic mutations), thereby providing potential for uncontrolled proliferation. Thus, "neoplastic cells" can include both dividing and non-dividing cells. For purposes of the invention, neoplastic cells include cells of tumors, neoplasms, carcinomas, sarcomas, leukemias, lymphomas, and the like. Of particular interest are central nervous system tumors, especially brain tumors. These include glioblastomas, astrocytomas, oligodendrogliomas, meningiomas, neurofibromas, ependymomas, Schwannomas, neurofibrosarcomas, etc. The invention can be utilized to target for oncolysis both benign and malignant neoplastic cells in the periphery and the brain. As used herein, the term periphery is intended to mean all other parts of the body outside of the brain. Thus, a peripheral tumor is intended to mean a tumor in a part of the body outside of the brain.

### The Transgene Carried by the Herpes Viral Mutant

In addition to having a tumor-specific or cell-specific promoter drive expression of the herpes γ34.5 gene (and possibly one or more additional mutations, such as, *e.g.*, RR, TK, UNG, or dUTPase, as described above), the viral mutants of the present invention can also carry a heterologous transgene.

The transgene can be a suicide gene, that is, a gene that encodes a gene product capable of activating a chemotherapeutic agent to its cytotoxic form, such as HSV-TK, CD, or cytochrome P450. In a preferred embodiment, the suicide gene is a cytochrome P450 gene.

By "gene product capable of converting a chemotherapeutic agent to its cytotoxic form" is meant a gene product that acts upon the chemotherapeutic agent to render it more cytotoxic than it was before the gene product acted upon it. Other proteins or factors may be required, in addition to this gene product, in order to convert the chemotherapeutic agent to its most cytotoxic form.

By "transgene encoding a gene product capable of converting a chemotherapeutic agent to its cytotoxic form" is meant a nucleic acid that upon expression provides this gene product.
"Cytotoxic" is used herein to mean causing or leading to cell death.
"Gene product" broadly refers to proteins encoded by the particular gene.
"Chemotherapeutic agent" refers to an agent that can be used in the treatment of neoplasms, and that is capable of being activated from a prodrug to a cytotoxic form. Preferably, the chemotherapeutic agents for use in the invention do not significantly inhibit replication of the viral mutant, which means that viral replication can occur at a level sufficient to lead to death of the infected cell and to propagate the spread of the virus to other cells.

The term "gene encoding cytochrome P450" means a mammalian cytochrome P450 gene such as, P450 2B1, P450 2B6, P450 2A6, P450 2C6, P450 2C8, P450 2C9, P450 2C 11, or P450 3A4. Each of these genes has been linked to activation of the anticancer drugs cyclophosphamide and ifosfamide (Clarke *et al*., *Cancer Res*. *49*:2344-2350 (1989); Chang *et al*., *Cancer Res*. *53*:5629-5637 (1993); Weber and Waxman, *Biochemical Pharmacology 45*:1685-1694 (1993)), and the cDNA sequences of these genes have also been published (Nelson *et al*., *DNA and Cell Biology 12*:1-51 (1993) and references cited therein; *Yamano et al*., *Biochem*. *29*:1322-1329 (1990); Yamano *et al*., *Biochem*. *28*:7340-7348 (1989)). Moreover, cytochrome P450 can also activate N-methyl cyclophosphamide (N-methyl CPA), methylchloropropylnitrosourea (MCPNU), and polymeric forms of CPA, ifosfamide, N-methyl CPA, and MCPNU. Polymeric forms of chemotherapeutic agents are discussed in Brem, *Biomaterials, 11:* 699-701 (1990); Buahin and Brem, *J. Neurooncol 26:* 103-110 (1995); Tamargo *et al*., *Cancer Res. 53*: 329-333 (1993); and Langer, *Ann. Biomed*. *Eng*. *23*: 101-111 (1995).

Persons of ordinary skill in the art should be able to utilize the method of the present invention with numerous other anticancer drugs that are activated by members of the cytochrome P450 family of enzymes (LeBlanc and Waxman, *Drug Metab*. *Rev*. *20*:395-439 (1989)), as well as with drug-metabolizing cytochrome P450 genes from other species (*e*.*g*., mouse, rabbit, hamster, dog, etc.) that are homologous to cytochromes P450 2B1, P450 2B6, P450 2A6, P450 2C6, P450 2C8, P450 2C9, P450 2C 11, or P450 3A4, and whose cDNA sequences are known (Nelson *et al*., *DNA and Cell Biology 12*:1-51 (1993)). In a particularly preferred embodiment, the gene encoding cytochrome P450 2B1 is used.

If the herpes viral mutant contains a suicide gene, the chemotherapeutic agent that is activated by the suicide gene should not significantly inhibit replication of the viral mutant so as to allow the viral mutant to kill tumor cells by viral oncolysis, as well as by delivery of the suicide gene. The use of a chemotherapeutic agent/transgene combination in which the chemotherapeutic agent, or its active metabolites, act instead by crosslinking DNA or by inhibiting DNA repair would not significantly inhibit replication of the viral mutant. Thus, such chemotherapeutic agent/transgene combinations are encompassed by the viral mutant and methods of the present invention.

Thus, a preferred chemotherapeutic agent/transgene combination is cytochrome P450 combined with CPA, ifosfamide, N-methyl cyclophosphamide, MCPNU, or polymeric forms of: CPA, ifosfamide, N-methyl cyclophosphamide and MCPNU. A more preferred chemotherapeutic agent/transgene combination is CPA/cytochrome P450 2B1.

Other chemotherapeutic agent/transgene combinations for use in the present invention include: CB 1954/*E*. *coli* nitroreductase (Friedlos *et al*., *Gene Ther. 5:* 105-112 (1998); Green *et al*., *Cancer Gene Ther. 4:* 229-238 (1997)); topoisomerase I or II inhibitors/enzyme with esterase-like activity, such as, *e.g.*, CPT-11/carboxylesterase (Jansen *et al*., *Int*. *J*. *Cancer 70:* 335-340 (1997); Danks *et al*., *Cancer Res. 58:* 20-22 (1998)); 4-ipomeanol/cytochrome P450 4B I (Verschoyle *et al*., *Toxicol*. *Appl*. *Pharmacol*. *123*: 193-198 (1993)); and 2-aminoanthracene/cytochrome P450 4B 1 (Smith *et al*., *Biochem*. *Pharmacol*. *50:* 1567-1575 (1995)).

The use of an alkylating agent such as CPA, while providing an anticancer effect, does not significantly inhibit viral protein synthesis or viral replication. The explanation for this finding may lie in the mode of action of these drugs. CPA's active metabolite, phosphoramide mustard (PM) produces interstrand and intrastrand crosslinks in cellular DNA. Maximum cytotoxicity to cellular DNA is usually achieved during mitosis when multiple DNA strand breaks occur at the cross-link sites (Colvin, in *Cancer Medicine*. eds. Holland *et al*., 1993. Lea and Fabiger, Philadelphia, pages 733-734). In contrast, non-mitotic, cross-linked viral DNA may be spared from extensive damage and may be thus be repaired more readily than cellular DNA.

Ganciclovir is one example of a chemotherapeutic agent that, when activated, inhibits viral replication. Although it has been demonstrated that the combination of hrR3 and ganciclovir provides a significant anticancer effect due to the conversion of ganciclovir by the viral thymidine kinase gene (Boviatsis *et al*., *Cancer Res. 54*: 5745-5751 (1994)), the converted ganciclovir molecules also inhibit viral replication. For this reason, use of TK/GCV may not be a preferred selection in this paradigm. Prodrug-activating enzymes, such as HSV-TK generate anticancer metabolites that act as "false" nucleotides, producing premature termination of replicating DNA strands. Therefore, these prodrug-activating enzymes would be expected to affect both viral and genomic DNA synthesis and would not be a good choice for use in the herpes viral mutants of the invention that contain a suicide gene.

Another advantage of using chemotherapeutic agents whose mechanism of action is the cross-linking of DNA or the inhibition of DNA repair enzymes is that these agents are effective against even cells in Go. Thus, for these agents to be effective in killing neoplastic cells, the targeted cells do not have to be actively dividing at the time that the drug is administered. This is a significant benefit for tumors in which a large percentage of cells are in Go.

One example of this type of tumor is the glioblastoma. For glioblastomas, the growth fraction, or the relative proportion of cells proliferating in the tumor at any one time, is only 30%, with the remaining 70% of cells being in G₀. These tumors are especially resistant to chemotherapeutic agents that target only actively dividing cells because, while the 30% of glioblastoma cells that are actively dividing contribute to the lethal progression of this tumor, 70% of the cells are in G₀ and may die or may re-enter the active cell cycle, Yoshii *et al*., *J*. *Neurosurg. 65*:659-663 (1986)). Thus, the 70% that are quiescent are responsible for the resistance of these tumors to chemotherapeutic agents that target actively proliferating cells.

This example demonstrates another advantage of the invention. The viral mutant and method of the present invention provide an advantage over therapies based on replication-conditional or replication-incompetent viral mediated oncolysis alone, in that those therapies will target only those cells that can complement the viral mutation. Whereas, although the viral mutant of the invention targets cells with elevated levels of E2F (primarily neoplastic cells) for replication in, and lysis, expression of the transgene and activation the chemotherapeutic agent provides active metabolites that can then diffuse to surrounding tumor cells. These metabolites can thereby kill even those surrounding tumor cells in G₀ (70% of the cells in a glioblastoma).

Accordingly, the invention finds particular use in the treatment of glioblastomas. The glioblastoma represents approximately 30% or 50% of all primary brain tumors and, despite surgery, chemotherapy, and radiation therapy, is almost universally fatal.

Due to local activation of the chemotherapeutic agent by the gene product of the gene carried by the herpes viral mutant, the method of the invention should allow more tumor toxicity at the same drug concentration, thus allowing for higher tumor doses without increasing toxicity to normal cells. Further, chemotherapeutic treatment of systemic tumor populations may also be improved by using the method of the present invention because lower doses of the drug may be possible by virtue of increased efficiency.

Furthermore, local activation of the chemotherapeutic agent provides another benefit. Some chemotherapeutic agents require activation or conversion to their active state in cells or organs in the periphery, however, often the active (cytotoxic) metabolites cannot cross the blood brain barrier, and thus are not effective against brain tumors. Thus, the method of the invention should allow treatment of brain tumors by these chemotherapeutic agents. One such chemotherapeutic agent is CPA. CPA is largely ineffective against central nervous system neoplasms as its conversion to DNA-alkylating, cytotoxic metabolites is restricted primarily to the liver and these metabolites do not readily cross the blood-brain barrier. However, the use of the viral mutant of the invention, engineered to carry a cytochrome P450 gene and applied to a brain tumor, would provide for local activation of CPA. Thus, in a preferred embodiment, a cytochrome P450 gene is utilized to sensitize central nervous tumor cells to the cytotoxic effects of cyclophosphamide (CPA).

In addition to a "suicide gene", the transgene can also encode a cytokine to stimulate or enhance a tumor-directed immune response. *See*, Blankenstein, T., *et al*., *J. Exp*. *Med*. *173*:1047-1052 (1991); Colombo, M.P., *et al*., *Cancer Metastasis Rev*. *16*:421-432 (1997); Colombo, M.P., *et al*., *Immunol*. *Today 15*:48-51 (1994)). Representative examples include tumor necrosis factor alpha (TNF-α), interferon-γ, interleukins (IL-2, IL-4), or granulocyte-macrophage colony stimulating factor (GM-CSF)).

The transgene could also encode a tumor suppressor gene, or any other tumoricidal gene known to those skilled in the art, such as diptheria toxin (Coll-Fresno, P.M., *et al*., *Oncogene 14*:243-247 (1997)), pseudomonas toxin, anti-angiogenesis genes, tumor vaccination genes, radiosensitivity genes, antisense RNA, or ribozymes (Zaia, J.A., *et al*., *Ann*. *N*.*Y*. *Acad*. *Sci*. *660:*95-106 (1992)).

Thus, in this embodiment of the invention, the herpes viral mutant, with a tumor-specific or cell-specific promoter driving expression of the γ34.5 gene, further comprises a transgene encoding a gene product capable of converting a chemotherapeutic agent to its cytotoxic form or any other tumoricidal transgene, as mentioned above. The transgene can be inserted in the viral genome in any location where it will be expressed. Preferred locations in the viral genome for the transgene are at the locus of the original γ34.5 deletion or anywhere in the herpes UL40 locus.

### Administration of the Herpes Viral Mutant

Exemplary candidates for treatment according to the presently claimed methods include, but are not limited to: (i) non-human animals suffering from neoplasms characterized by a tumor-specific promoter or cell-type specific promoter; (ii) humans suffering from neoplasms characterized by a tumor-specific promoter or cell-type specific promoter; (iii) humans or non-human animals in need of eradication of a particular cell population.

By "neoplastic cells" is intended cells whose normal growth control mechanisms are disrupted (typically by accumulated genetic mutations), thereby providing the potential for uncontrolled proliferation. The term is intended to include both benign and malignant neoplastic cells in both the central nervous system and the periphery. As used herein, the term "periphery" is intended to mean all other parts of the body outside of the brain or spinal cord.

For purposes of the invention, neoplastic cells include cells of tumors, neoplasms, carcinomas, sarcomas, papillomas, leukemias, lymphomas, and the like. Of particular interest are solid tumors that may arise in any organ or tissue of the mammalian body.

Malignant brain tumors, include astrocytoma, oligodendroglioma, meningioma, neurofibroma, glioblastoma, ependymoma, Schwannoma, neurofibrosarcoma, and medulloblastoma.

Preferentially, the treatment will be initiated by direct intraneoplastic inoculation. For tumors in the brain, MRI, CT, or other imaging guided stereotactic techniques may be used to direct viral inoculation, or virus will be inoculated at the time of craniotomy. For patients attempting to eradicate a particular cell population, the vector would be inoculated into the tissue of interest.

Generally, methods are known in the art for viral infection of the cells of interest. For example, the viral mutant can be injected into the host at or near the site of neoplastic growth, or administered by intravascular inoculation. Typically, the viral mutant would be prepared as an injectable, either as a liquid solution or a suspension; a solid form suitable for solution in, or suspension in, liquid prior to injection may also be prepared. The preparation also may be emulsified. The active ingredient is preferably mixed with an excipient which is pharmaceutically-acceptable and compatible with the active ingredient. Suitable excipients are, for example, water, saline, dextrose, glycerol, ethanol, or the like and combinations thereof. In addition, if desired, the preparation may contain minor amounts of auxiliary substances such as wetting or emulsifying agents, pH-buffering agents, adjuvants or immunopotentiators which enhance the effectiveness of the viral mutant (*See*, *Remington's Pharmaceutical Sciences*, Gennaro, A.R. *et al*., eds., Mack Publishing Co., pub., 18th ed., 1990). Examples of non-aqueous solvents are propylene glycol, polyethylene glycol, vegetable oil and injectable organic esters such as ethyloleate. Aqueous carriers include water, aqueous solutions, saline solutions, parenteral vehicles such as sodium chloride, Ringer's dextrose, etc. Intravenous vehicles include fluid and nutrient replenishers. Determining the pH and exact concentration of the various components of the pharmaceutical composition is routine and within the knowledge of one of ordinary skill in the art (*See Goodman and Gilman's The Pharmacological Basis for Therapeutics*, Gilman, A.G. *et al*., eds., Pergamon Press, pub., 8th ed., 1990).

Additional formulations which are suitable include oral formulations. Oral formulations include such typical excipients as, for example, pharmaceutical grades of mannitol, lactose, starch, magnesium stearate, sodium saccharine, cellulose, magnesium carbonate and the like. Oral compositions may take the form of tablets, pills, capsules, sustained release formulations or powders and contain 10%-95% of active ingredient, preferably 25-70%.

The dosage of the viral mutant to be administered, in terms of number of treatments and amount, depends on the subject to be treated, the capacity of the subject's immune system to synthesize antibodies, and the degree of protection desired. Precise amounts of active ingredient required to be administered depend on the judgment of the practitioner and are peculiar to each individual. For the most part, the virus is provided in a therapeutically effective amount to infect and kill target cells.

### Methods for Selectively Killing Neoplastic Cells

The present invention also provides a method for selectively killing neoplastic cells that overexpress a known tumor-specific promoter using the herpes viral mutants described above, comprising: infecting said neoplastic cells with said herpes viral mutant, said viral mutant comprising: (a) a deletion or inactivating mutation in a gene encoding γ34.5; and (b) an insertion of at least one copy of said γ34.5 gene under the transcriptional control of said tumor-specific promoter, such that said promoter drives expression of said γ34.5 gene; and selectively killing said neoplastic cells.

Of course, in the herpes viral mutant used in the above method, there may be more than one specific endogenous deletion or inactivating mutation of a herpes viral gene, in addition to the γ34.5 gene. These include deletions in the gene that encodes ribonucleotide reductase (RR), or more particularly the large subunit of RR. Alternatively, the gene encoding RR encodes the small subunit. Any other herpes viral genes may also be deleted, such as, e.g., thymidine kinase (TK), uracil DNA glycosylase (UNG), or dUTPase. These viral genes are preferred as the mammalian homologues of these genes are often up-regulated in cells with elevated levels of E2F, such as neoplastic cells, and thus can complement the deleted viral enzyme, thereby promoting selective replication in those cells.

Exemplary tumor-specific promoters are described above, and include, for example, CEA, AFP, tyrosinase, and PSA. Tumor cells are also known to overexpress particular oncogenes, so that cells with upregulated gene expression can be targeted using promoter elements of such genes. B*-myb*, C-*myb*, c-*myc*, c-*kit*, and the c-*erb*B2 oncogene are some representative examples of these types. The B-*myb* promoter (*see*, Lyon, J., *et al*., *Crit*. *Rev*.*Oncogenesis 5*:373-388 (1994) contains a consensus E2F binding site, is strictly regulated in cycling cells, and is in fact repressed in G₀ (Lam, E.W. and Watson, R.J., *EMBO J*. *12*:2705-2713 (1993); Lam, E.W., *et al*., *Gene 160*:277-281 (1995); Bennett, J.D., *et al*., *Oncogene 13*:1073-1082 (1996)). Accordingly, the B-*myb* promoter is a particularly preferred tumor-specific promoter. In a particularly preferred embodiment of this method, the viral mutant used in the method is Myb34.5.

Any cancer type having a well-characterized promoter would find use in the method of the invention. Examples of such promoters can be found in Table 1 of Clary, B.M., *et al*., Cancer Gene Therapy 7:565-574 (1998); Table I of Spear, M.A., *Anticancer Research 18*:3223-3232(1998); Table 2 of Walther, W. and Stein, U., *J*.*Mol*.*Med*. *74*:379-392(1996); and Dachs, G.U., *et al*., *Oncol*. *Res*. *9*:313-325 (1997)).

Most if not all of the gene sequences of the tumor-specific promoters described above are available from the GenBank Sequence Database.

In addition, the invention provides the above method for selectively killing neoplastic cells, wherein said herpes viral mutant further comprises a transgene, wherein the transgene is a suicide gene, a cytokine gene, or any tumoricidal gene. If the transgene is a suicide gene, then the method further comprises contacting the neoplastic cells with a chemotherapeutic agent capable of being activated by said suicide gene and selectively killing the neoplastic cells. The preferred suicide gene is cytochrome P450. P450 2B1 is particularly preferred. Alternatively, the cytochrome P450 encoded is P450 2B6, P450 2A6, P450 2C6, P450 2C8, P450 2C9, P450 2C11, or P450 3A4. In addition, the chemotherapeutic agent is preferably a member of the oxazosphorine class, particularly cyclophosphamide, ifosfamide, N-methyl cyclophosphamide, methylchloropropylnitrosourea, polymeric cyclophosphamide, polymeric ifosfamide, polymeric N-methyl cyclophosphamide, or polymeric methylchloropropylnitrosourea.

Another embodiment of the invention is a pharmaceutical composition containing the foregoing viral mutants, wherein this composition may also contain one or more pharmaceutically acceptable excipients.

### Methods for Selectively Eliminating Target Cell Populations

Another embodiment of the present invention is a method for selectively eliminating a target cell population that overexpresses a known cell-specific promoter using the herpes viral mutants of the invention, comprising: infecting said target cells with said herpes viral mutant, said viral mutant comprising: (a) a deletion or inactivating mutation in a gene encoding γ34.5; and (b) an insertion of at least one copy of said γ34.5 gene under the transcriptional control of said cell-specific promoter, such that said promoter drives expression of said γ34.5 gene; and selectively eliminating a target cell population.

By "selectively eliminating a target cell population" is intended to include a significant reduction in the number of target cells versus non-target cells, as well as the complete or near complete elimination of target cells.

Of course, in the herpes viral mutant used in the above method, there may be more than one specific endogenous deletion or inactivating mutation of a herpes viral gene, in addition to the γ34.5 gene. These include deletions in the gene that encodes ribonucleotide reductase (RR), or more particularly the large subunit of RR. Alternatively, the gene encoding RR encodes the small subunit. Any other herpes viral genes may also be deleted, such as, *e*.*g*., thymidine kinase (TK), uracil DNA glycosylase (UNG), or dUTPase.

Exemplary cell-specific promoters include the following: vascular endothelial growth factor (VEGF) receptor (flk1) promoter expressed in endothelial cells (Kappel *et al*. *Blood 93*: 4282-4292 (1999); insulin promoter expressed in beta cells of the pancreas (Ray *et al*., *J*. *Surg*. *Res*. *84*: 199-203 (1999); gonadotropin-releasing hormone receptor gene expressed in cells of the hypothalamus (Albarracin *et al*., *Endocrinology 140*: 2415-2421 (1999); matrix metalloproteinase 9 promoter, expressed in osteoclasts and keratinocytes (Munant *et al*., *J*. *Biol*. *Chem. 274:* 5588-5596 (1999); Parathyroid hormone receptor expressed in bone cells (Amizuma *et al*., *J*. *Clin. Invest. 103*: 373-381 (1999); dopamine beta-hydroxylase promoter expressed in noradrenergic neurons (Yang *et al*., *J*. *Neurochem*. *71*: 1813-1826 (1998).

Examplary applications of this embodiment include the following:
1) Treatment options to eliminate a noxious cell population: For example, in conditions where there is exuberant neovascularization of blood vessels, such as cerebral Moya-Moya disease, use of the flk 1 receptor promoter to drive gamma 34.5 gene expression would allow for selective elimination of the blood vessels causing this disease. Another example is in conditions where there is extensive bone remodeling and elimination of bone, such as osteoporosis, the use of the matrix metalloproteinase 9 or the parathyroid hormone receptor to drive expression of gamma 34.5 would eliminate bone osteoclasts from further remodeling of bone.
2) To study developmental processes: In order to study the effect of elimination of a cell population on developmental processes, one could use, for example, the dopamine-beta-hydroxylase promoter to eliminate the noradrenergic neurons and then study the effect on animal development.

The following examples are offered by way of illustration, not by way of limitation.

### Example 1

### Introduction

Deletion of the γ34.5 gene encoding for virulence markedly reduces cytotoxicity mediated by herpes simplex virus (HSV). To target lytic virulence to tumors, the inventors created a herpes simplex virus (HSV1) mutant designated Myb34.5. This viral mutant is characterized by deletions in ICP6 (also known as UL39 or ribonucleotide reductase) and of the two endogenous copies of the γ34.5 gene (RL1) and by reintroduction of one copy of γ34.5 under control of the E2F-responsive, cellular B-*myb* promoter.

On direct intracerebral inoculation in mice, Myb34.5 remained as avirulent as a γ34.5 mutant virus. However, its oncolytic efficacy against a variety of human glioma cells in culture and *in vivo* was similar to that of a single ICP6 mutant strain that possesses a wild-type γ34.5 gene. This combination of antitumor efficacy and retained neuroattenuation suggests that cell cycle-regulated promoters can be used to target HSV-1 virulence toward tumors, while maintaining the desirable neuroattenuated phenotype of a γ34.5 mutant.

### Methods and Materials

**Plasmids and Viruses -** HSV strain F (wild-type) was acquired through the ATCC (Manassas,VA). Mutant virus R3616 (Chou, J., *et al*., *Science 250*:1262-1266 (1990)) (containing 1000bp BstEII-StuI deletions within both γ34.5 loci) was kindly provided by Dr. B. Roizman, University of Chicago. Mutant virus hrR3 (Goldstein, D.J. and Weiner, S.K., *J*. *Virol*. *62*:196-205 (1988)) (kindly provided by S. Weller, University of Connecticut) contains an *E*. *Coli* lacZ cDNA inserted into the UL39 locus. The mutant virus MGH1 is characterized by insertion of the *Escherichia coli* lacZ cDNA into the UL39 locus and deletions of both γ34.5 loci, and it was constructed by recombination of the ICP6-lacZ region of hrR3 into the viral mutant R3616 (Kramm, C.M., *et al*., *Hum*. *Gene Ther*. *8*:2057-2068 (1997)). Plasmid pKX-BG3, which contains the lacZ gene within a 2.3 kb XhoI region of ICP6 (KOS origin, see, Goldstein,D.J. and Weller, S.K., *J*. *Virol*. *62*:2970-2977 (1988)), was provided by S. Weller, as was plasmid pKpX2, which contains 2.3 kb of the ICP6 (UL39) gene. Plasmid pBGL34.5, containing the entire γ34.5 coding sequence, was provided by Xandra Breakefield and Peter Pechan (MGH). The B-*myb* promoter was excised as a KpnI-HindIII fragment from plasmid pBGL2myb (kindly provided by Dr. R. Watson, Ludwig Institute for Cancer Research, UK) and directionally cloned upstream of γ34.5.

**Engineering of Myb34.5 and of a Myb34.5 revertant** - The plasmid used for the engineering of Myb34.5 by homologous recombination into MGH1 was designed to replace the *lacZ* cDNA in MGH1 in its entirety and delete an additional 888 nucleotides of ICP6 (UL39) sequence. Specifically, the recombining plasmid (pKpX2-myb34.5) was engineered as follows. The full-length γ34.5 cDNA was excised as an *Nco*I-*Sacl* fragment from pBGL34.5, it was blunt-ended, and then it was subcloned into pBSKII (Stratagene, La Jolla, Calif.) to generate plasmid pBS34.5. The B-*myb* promoter was excised as a *Kpn*I-*Hin*dIII fragment from pBGL2myb and directionally cloned upstream of γ 34.5 in pBS34.5. The resulting expression cassette, containing the B-*myb* promoter upstream of the γ34.5 cDNA, was excised as *Kpn*I-*Xba*I fragment, was blunt-ended, and was then subcloned into the *Nru*I sites of pKpX2. Through this process, the intervening *Nru*I-*Nru*I fragment within UL39 was deleted. The resulting plasmid, pKpX2-myb34.5, was then linearized with *Sca*I and contransfected with MGH 1 viral DNA into Vero cells at various molar ratios with Lipofectamine (Gibco, Gaithersburg, MD). Virus progeny was harvested 5 to 7 days following transfection when cytopathic effects were evident. This progeny was released from cells through three cycles of freeze-thawing, and it was then plated onto a monolayer of Vero cells. After overlayering the monolayer with agarose, incubation at 37 °C in an atmosphere containing 5% carbon dioxide was performed. Plaques were then stained with 5-bromo-4-chloro-3-indolyl-β-D-galactopyranoside (X-Gal). Colorless plaques were selected as potential recombinants. These isolates underwent three rounds of plaque purification before having their genetic identity tested by Southern blot analysis. A Myb34.5 revertant (MybRevt) was engineered by using Myb34.5 as the parental strain and pKX-BG3 as the plasmid for homoglous recombination of the *lacZ* cDNA back into the ICP6 locus and deletion of the B-*myb*/γ34.5 expression cassette.

**Southern Blot Analysis -** Viral DNAs were isolated after cell lysis of infected Vero cells with SDS/proteinase K, repeated phenol-chloroform extraction and ethanol precipitation. DNA was digested with appropriate restriction endonucleases (New England Biolabs, Beverly MA), separated by agarose electrophoresis, and transfered to a nylon membrane (Amersham Corp., Arlington Heights, Illinois). Probes included the HindIII-Xbal ICP6 fragment from pKpX2, the BstEII-BbsI fragment from pBSKγ34.5, and a BbsI fragment of lacZ from pKX-BG3. Probe labeling and hybridizations were performed using the ECL chemiluminescence system (Amersham) according to the manufacturer's protocol.

**Cell culture studies -** All cells were cultured at 37°C in an atmosphere containing 5% carbon dioxide in Dulbecco's minimal essential medium (DMEM) supplemented with 10% fetal calf serum, 100U of penicillin/ml, and 10 µg of streptomycin/ml. Host protein synthesis shutoff studies were performed by infecting cells with viral strains for 16 hours. Cells were then placed in methionine-free medium for 10 minutes, then labeled using ³⁵[S]-methionine (New England Nuclear, Boston MA) for 90 minutes. Cells were then washed with phosphate buffered saline (PBS) pH 7, solubilized, subjected to SDS polyacrylamide gel electrophoresis, transferred to a nitrocellulose membrane, and subjected to autoradiography. Protein concentrations were calculated with a commercially available kit (Bio-Rad, Hercules CA). Infected cell polypeptides (ICP) were labeled, as previously published (Morse, L.S., *et al*., *J*. *Virol*. *26*:389-410 (1978)). Human glioblastoma cell lines U87, U373, T98G, and U343; rat gliosarcoma 9L cells; human neuroblastoma SKNSH cells and Vero (African green monkey) cells were obtained from the American Type Culture Collection (Manassas, VA) and cultured with DMEM or minimal alpha essential medium (Gibco) supplemented with 10% serum and antibiotics. Primary mouse fetal striatal neurons (embryonic stage 18) (kindly provided by M. Schwarzchild, Massachusetts General Hospital, Charlestown, MA) were isolated using published procedures (Schwarzschild, M.A., *et al*., *J*. *Neurosci*. *17*:3455-3466 (1997)).

**Animal studies -** Nude (nu/nu) mice were obtained from the Cox 7 breeding facility, Massachusetts General Hospital (MGH). BALB/C mice were obtained from Charles River Laboratories (Wilmington, MA). Subcutaneous tumors were obtained by injection of 2 x 10⁵ cells in the flanks of athymic mice (five animals per group for 9L gliosarcoma cells, and six animals per group for human U87ΔEGFR glioma cells). Fourteen (for 9L) or ten (for U87ΔEGFR) days after tumor implantation, animals with similar tumor volumes were randomly divided, and various viral strains were injected intratumorally at 5 x 10⁷ PFU/dose in 100 ul volumes on days 1,3,5, and 7. Animals were euthanized at day 33 (9L) or day 34(U87ΔEGFR). Tumor volumes were measured with external calipers, as previously described (Wei, M.X., *et al*., *Hum. Gene Ther. 5*:969-978 (1994)). For neurotoxicity experiments, BALB/C mice were stereotactically injected in the right frontal lobe (depth 3mm) with 10 µl volumes of virus at different dilutions, up to the highest stock titers obtainable. Animals were checked daily for 28 days. All animal studies were performed in accordance with guidelines issued by the MGH Subcommittee on Animal Care. Viral inoculation and care of animals harboring viruses were performed in approved viral vector rooms.

### Results

**Genetic engineering of Myb34.5 -** The multiply mutated virus Myb34.5 was constructed by recombining a B-*myb* promoter/γ34.5 construct into the UL39 (also known as ICP6 or RR) locus of MGH1. MGH1 (Kramm, C.M., *et al*., *Hum*. *Gene Ther*. *8*:2057-2068 (1997)) was generated by recombining a *lac*ZcDNA into the ICP6 locus of the γ34.5 deletion mutant R3616 (Chou, J., *et al*., *Science 250*:1262-1266 (1990)). Figure 1A provides a schematic of the DNA structure of Myb34.5. This structure was confirmed by restriction endonuclease mapping (data not shown), Southern blot hybridization (Figs. 1B to 1D), and sequence analysis (data not shown) of the junctions between UL39 and the B-*myb* promoter/γ34.5 expression cassette. To show the deletion of *lacZ* in Myb34.5, *Xho*I-digested Myb34.5 DNA was hybridized to probes containing either ICP6 (Fig. 1B) or *lacZ* sequence (Fig. 1C). As expected, the parental virus, MGH1, contained a 9.0 kb *Xho*I ICP6*-lacZ* fragment (Kramm, C.M., *et al*., *supra*.) that hybridized to an ICP6 probe (Fig. 1B). Homologous recombination led to the deletion of lacZ and additional ICP6 sequence and insertion of the B*-myb* promoter/γ34.5 sequence. This is evident by hybridization of the ICP6 probe to a 6.7 kb fragment in Myb34.5 DNA (Figure 1B). Hybridization with a *lacZ* probe revealed the absence ofhybridizing fragments in digested DNA from Myb34.5 and the presence of the expected 9.0 kb hybridizing fragment in digested DNA from MGH1 (Fig. 1C). To confirm that Myb34.5 possessed a reintroduction of the γ34.5 gene, BamHI-digested viral DNA was hybridized with a BstE11-Bbs γ34.5 fragment (internal to the deleted regions). This demonstrated a ladder of hybridizing bands that is typically observed with the wild-type F strain. As discussed in the work of Chou *et al*. (1991), *supra*, γ34.5 maps in *Bam*HI S and SP fragments, forming a characteristic ladder of bands at 500 bp increments, which are a consequence of a variable number of *a* sequences in the repeats flanking the unique sequences of the long component. This ladder is observed in the Southern blot for the F strain (lane I of Fig. I D), where the top hybridizing bands represent the *Bam*HI SP fragment, formed by the fusion of the terminal *Bam*HI S fragment with *Bam*HI P, while the lower hybridizing bands represent the *Bam*HI S fragment (Chou, J., *et al*., *Science 250*:1262-1266 (1990)).

In R3616 and its derived viruses, MGH1, Myb34.5, and Myb34.5Revt, a similar ladder of hybridizing bands whose molecular size was decreased by approximately 1 kb (the size of the internal deletion of γ34.5 in R3616) would be expected if a full-length γ34.5 cDNA probe were employed for hybridization. In fact, in the work of Chou *et al*. (Chou, J., *et al*., *Science 250*:1262-1266 (1990)), this pattern of hybridization is evident for R3616, and in the work of Kramm *et al*. (Kramm, C.M., *et al*., *Hum*. *Gene Ther*. *8*:2057-2068 (1997)), this pattern of hybridization is evident for MGH1, However, for the Southern analysis shown in Fig. 1D, a *Bst*EII-*Bbs* γ34.5 fragment that is internal to the 1 kb deleted fragment of the γ 34.5 gene of R3616, MGH1, Myb34.5, and Myb34.5Revt was employed as a probe. Therefore, no hybridizing bands are observed for MGH1 (Fig. 1D, lane 2) and Myb34.5Revt (Fig. 1D, lane 4), while a single 5.3 kb hybridizing fragment is observed for Myb34.5 (Fig. 1D, lane 3), corresponding to the γ34.5 gene, reintroduced into the ICP6 locus.

In order to demonstrate that the altered phenotype of Myb34.5 was the result of the B-myb/γ34.5 insertion, a revertant (marker-rescued) virus was also engineered, and designated MybRevt. This was achieved by homologous recombination, with Myb34.5 as the parental strain, and linearized pKX2-BG3 as the recombining plasmid. This plasmid contains the lacZ/ICP6 insertion and was used to create hrR3, the source of the ICP6::lacZ fusion region in MGH1 (Kramm, C.M., *et al*., *Hum*. *Gene Ther*. *8*:2057-2068 (1997); Goldstein, D.J. & Weller, S.K., *J*. *Virol*. *62*:2970-2977 (1988)). The MybRevt revertant demonstrated a pattern on Southern hybridization to the ICP6 (Fig. 1B), *lacZ* (Fig. 1C), and γ34.5 (Fig. I D) probes, that was identical to that shown by MGH1, the parent strain of Myb34.5.

**Functional expression of γ34.5** - To confirm that Myb34.5 produced functional γ34.5 protein, human SKNSH neuroblastoma cells were infected with a variety of viral strains. Figure 2 shows that, as expected, MGH1 and the revertant virus (MybRevt) failed to prevent the infected cell response consisting of shut-off of protein synthesis, which is characteristic of intact γ34.5 function (Chou, J., 1992, *supra*). However, Myb34.5 and other strains with intact γ34.5 (wild-type F and hrR3) prevented the infected cell response (shut-off of protein synthesis), thus leading to viral protein production.

Additional evidence that Myb34.5 expressed functional γ34.5 protein was provided by assessment of viral replication in the U373 glioblastoma cell line which has been previously noted to restrict replication of γ34.5 mutant HSV strains (Mohr. I. and Bluzman, Y., *EMBO J*. *15*:4759-5766 (1996)). After infecting 5 x 10⁵ cells at a MOI of 1.0 and harvesting viral output 48 hours later, Myb34.5 yields were similar to those of wild-type F strain (1.1 x 10⁷ PFU vs. 5.0 x 10⁷ PFU, respectively). In contrast, yields of MGH1 (3.3 x 10⁴ PFU) or MybRevt (3.4 x 10⁴ PFU, averages of triplicate experiments) were significantly less. The ability of Myb34.5 to efficiently replicate in this non-permissive line, in contrast to that of MGH1 or MybRevt, indicates that the encoded γ34.5 in Myb34.5 was functional.

**Neurotoxicity Studies -** One important characteristic of any replication-competent HSV strain is its level of neurovirulence, which can be assessed both *in vitro* and *in vivo.* The ability of the Myb34.5 virus to replicate in primary neuronal cultures was measured in comparison to the F, hrR3, MGH1, and MybRevt strains. Murine fetal striatal neurons were infected, and viral yields were assessed by plaque assay on Vero cells (which do not require γ34.5 for efficient viral replication). While the wild-type F strain demonstrated vigorous replication in neurons, all the mutant strains, including Myb34.5, demonstrated minimal viral replication (*See*, Table 1).

**TABLE 1**

| **Viral Replication in Primary Neuronal Culture*** | |
|---|---|
| HSV Strain | Mean (SEM) Viral Output (PFU) |
| F (wt) | 1.0 x 10⁷ (1.4 x 10⁶) |
| hrR3 | 4.1 x 10³ (4.2 x 10²) |
| MGH1 | 1.4 x 10³ (73) |
| Mybγ34.5 | 6.0 x 10³ (4.3 x 10²) |
| MybRevt | 1.5 x 10³ (74) |

| | |
|---|---|
| *A total of 2.5 x 10⁵ murine fetal (day 18) striatal neurons were harvested and plated as previously described (Chase, M., *et al*., *Nat*. *Biotechnol*. *16*:444-448 (1998)). Two days after plating, cells were infected at MOI of 0.1 with wild-type (strain F) and mutant viral strains in duplicate experiments. Twenty-four hours after infection, cells and supernatant were harvested and subjected to freeze-thaw cycles to liberate viral particles. Viral output was determined by plaque assay on Vero cells. Standard errors (SEM) are enclosed in parentheses. Viral outputs of the four mutant viral strains (hrR3, MGH1, Myb34.5, and MybRevt) were not statistically different between groups (t-test, p>0.3). | |

In the *in vivo* setting, direct intracerebral inoculation of Myb34.5 in BALB/C mice demonstrated that the virus remains markedly neuroattenuated relative to wild-type HSV. Table 2 shows that Myb34.5's LD₅₀ was 2.7 x10⁷PFU, at least 3 logarithmic units higher than that of F strain (wild-type). The γ34.5 mutants (MGH1, R3616, and MybRevt) do not grow well, and achievement of titers of > 10⁹ PFU/ml is prohibitive without large-scale production. This limited the ability to estimate accurately the LD₅₀ for these mutants in these experiments. For comparative purposes, one of six mice perished when inoculated intracerebrally with 10⁷ PFU of Myb34.5, while zero of six mice perished when inoculated with the same amount of MGH1. These findings confirmed that reintroduction of γ34.5 under the control of the B-*myb* promoter produced minimal neurovirulence.

**TABLE 2**

| **Comparative ability of wild-type and mutant viral strains to cause death after intracerebral inoculation into mice*** | | |
|---|---|---|
| **Virus in inoculum** | **Description** | **LD**_{**50**} **(PFU)** |
| HSV-1 (F) | Wild-type | 1.0 x 10⁴ |
| hrR3 | lacZ insertion in RR | 1.3 x 10⁶ |
| R3616 | γ34.5 mutant | >1 x 10⁷** |
| MGH1 | γ34.5/RR double mutant | >1 x 10⁷** |
| Myb34.5 | RR/myb34.5 | 2.7 x 10⁷*** |

| | | |
|---|---|---|
| *Serial dilutions of virus in serum free media (volume 10 µl) were stereotactically injected into the right hemisphere of three week old female BALB/C mice (Charles River Laboratories, Wilmington, MA, 6 animals per PFU level). Animals were inspected daily for 28 days for mortality, and the LD₅₀ in PFU was calculated using a proportional distance model. Double asterisks | | |
| (**) for R3616 and MGH1 denote that no deaths occurred atthe listed dose, which was the highest attainable for these strains in the given injection volume. This is because titers of > 10⁹ PFU/ml could not be technically achieved with these mutants in growing Vero cells without industrial-scale production and intracerebral injections were limited to a maximum volume of 10 µl. Triple asterisks | | |
| (***) indicate that in parallel experiments, one death per six animals occurred at a dose of 10⁷ PFU. Since titers of 5 x 10⁹ to 1 x 10¹⁰ PFU/ml could be achieved with Myb34.5 in rapidly dividing Vero or tumor cells, a more accurate LD₅₀ could be measured. | | |

**Regulation of γ34.5 gene expression in arrested and cycling cells** - In order to further assess the behavior of Myb34.5 in quiescent versus cycling cells, primary human fibroblasts were plated and cell-cycle arrested with 20µM lovastatin, which has been shown not to interfere with herpes virus replication (Schang, L.M., *et al*., *J*. *Virol*. *72*:5626-5637 (1998)). In arrested primary fibroblasts, MGH1, Myb34.5, and MybRevt demonstrated minimal viral replication relative to F strain, at levels 1 logarithmic unit (PFU) lower than the single RR mutant hrR3 (Figure 3). In contrast, in the presence of serum, hrR3 and Myb34.5 demonstrated marked induction of replication, while MGH1 and MybRevt did not. These results suggested that: 1) γ34.5 is required for efficient replication in this nontransformed cell type, and 2) the B-*myb* promoter functions to allow efficient replication of Myb34.5 in cycling cells. It is also notable that Myb34.5 exhibited a greater differential in the production of viral progeny in quiescent versus cycling cells than that of hrR3 (3 versus 2 logarithmic units) and that its viral production in quiescent cells was the same as that of the γ34.5 mutant viruses.

**Comparative studies on oncolytic effects -** To determine the oncolytic efficiency of Myb34.5, five different glioma cell lines (9L, U87, U87ΔEGFR, T98G, and U343) were mock infected (no virus) or infected with a panel of viral strains including F strain, MGH 1, Myb34.5, and MybRevt. Surviving cells were counted 48 hours later and expressed as a percentage of cells surviving on mock-treated plates. In published studies of rat 9L gliosarcoma cells (Kramm, C.M., *et al*., *Hum*. *Gene Ther*. *8*:2057-2068 (1997)) and in unpublished experiments on human glioma cells U343 and T98 (Qureshi, N. and Chiocca, E.A., unpublished data), it had preliminarily been shown that tumor cell killing by MGH 1 was similar to that by R3616 for two human glioma lines (Table 3), and thus the latter mutant was excluded from further analysis.

In all tumor cell lines tested, Myb34.5 demonstrated greater oncolytic efficiency *in vitro* than did the the parental strain MGH 1, and for some tumor cell lines its oncolytic efficacy approached that ofthe wild-type virus (Table 3). These findings thus showed that the oncolytic effect of Myb34.5 was greater than that of the γ34.5 mutant viruses (MGH 1, MybRevt, and R3616, whose killing efficacy closely replicates that of MGH1).

**TABLE 3**

| ***In vitro* glioma cell killing by wild-type and mutant HSV strains *** | | | | |
|---|---|---|---|---|
| **Cell line** | **% Survival (SEM) After Infection with HSV Strain** | | | |
| | **F** | **MGH1**** | **Myb34.5** | **MybRevt** |
| U87 | 2.9(0.7) | 52.(2.0) | 19.5(1.5) | 51 (2.5) |
| 9L | 23.7(1.7) | 60 ( 1.2) | 29(1.4) | 59.5(1.4) |
| U343 | 19 (1.1) | 35.7(1.6) | 26.3 (1.1) | 32.3(3.0) |
| T98G | 26.8(0.8) | 38.5(1.0) | 29.3(1.7) | 40(1.2) |
| U87ΔEGFR | 15 (0.4) | 48 (0.3) | 10 (0.6) | 50 (0.7) |

| | | | | |
|---|---|---|---|---|
| *Human (U87MG, U343, T98G, and U98ΔEGFR) and rat (9L) glioma-derived cell lines were plated at 5 x 10⁵ cells / 60 mm diameter plate and subsequently infected at an MOI of 0.1 with the strains noted. Oncolytic effect is reflected by cell survival at 48 hours post infection, expressed as a percentage of the number of surviving cells from triplicate, non-infected control plates. Values represent averages (SEM in parentheses) of triplicate experiments. Differences in killing of tumor cells by Myb34.5 versus MGH 1 or MybRevt were statistically significant. For example, for U87 glioma cells, *P* was <0.001 (one-way analysis of variance with Tukey pairwise multiple comparison procedures). | | | | |
| **Killing for R3616 was not included in this particular set of experiments because it is relatively similarto that observed with MGH1. This was shown by Kramm *et al*. (Kramm, C.M., *et al*., *Hum*. *Gene Ther*. *8*:2057-2068 (1997)) for rat 9L gliosarcoma cells as well as in a number of previously unpublished experiments performed at a different time from the present experiment. For example, for human U343 glioma cells infected with MGH1 or R3616 cells at an MOI of 0.1, survival rates were 54 and 50%, respectively, at 2 days. For human T98 cells infected with MGH or R3616 at an MOI of 0.1, survival rates were 40 and 38%, respectively. | | | | |

***In vivo* anticancer effects -** The *in vivo* anticancer effects of Myb34.5 were then determined. After establishing rat 9L gliosarcoma (Fig. 4A) or human U87dEGFR glioma (Fig.4B) tumors in the flanks of athymic mice, intraneoplastic inoculation with each virus was performed. 9L tumor growth, as assessed by mean tumor volumes, was significantly reduced by Myb34.5 treatment compared to control animals, with inhibition quantitatively similar to that after hrR3 treatment (Fig.4A). For U87ΔEGFR, a human glioma which expresses a common truncated EGF receptor (Nagane, M., *et al*., *Cancer Res. 56*:5079-5086 (1996); Huang, H.S., *et al*., *J*. *Biol*. *Chem*. *272*:2927-2935 (1997); Nishikawa, R., *et al*., *Proc*. *Natl*. *Acad*. *Sci*. *USA 91*:7727-7731 (1994)), all strains significantly inhibited growth, with hrR3 and Myb34.5 producing 3 of 6 complete regressions, while MGH1 and MybRevt produced 2 of 6 regressions (to no visible tumor, Fig. 4B).

Although a cursory analysis of Fig. 4B may lead to the conclusion that there were no significant differences in the growth of human U87ΔEGFR tumors treated with the γ34.5 mutants (MGH1 and MybRevt) versus the γ34.5⁺ viruses (hrR3 and Myb34.5), review of the actual tumor volumes at the 34-day point does reveal the presence of a significant difference (Table 4). These results thus showed that Myb34.5's *in vivo* oncolytic effects paralleled those of the single RR mutant and were superior to those of the γ34.5 mutants.

**TABLE 4**

| **Volumes of human U87ΔEGFR tumors after treatment**^{***a***} | | | | | |
|---|---|---|---|---|---|
| | **Tumor vol (mm**^{**3**}**) after:** | | | | |
| **Animal no.** | **Mock** | **Infection with viral mutant** | | | |
| | **infection** | **HrR3** | **MGH1** | **Myb34.5** | **MybRevt** |
| 1 | 11,880 | 0 | 3,960 | 4,000 | 221 |
| 2 | 19,530 | 32 | 0 | 0 | 561 |
| 3 | 15,592 | 15 | 0 | 132 | 0 |
| 4 | 10,626 | 0 | 1,300 | 0 | 3,420 |
| 5 | 10,584 | 0 | 1,914 | 0 | 3,344 |
| 6 | 15,620 | 3,366 | 900 | 405 | 0 |
| Median^{*b*} | 13,736 | 7.5 | 1,100 | 66 | 391 |
| Confidence range (25-75%) | 10,626-15,620 | 0-32 | 0-1,914 | 0-405 | 0-3,344 |

| | | | | | |
|---|---|---|---|---|---|
| ^{*a*}Details of experimental procedures are provided in the legend to Fig. 4. Results are from the 34-day time point. | | | | | |
| ^{*b*}The differences in the median values among the treatment groups were statistically different (*P* = 0.004 [Kruskal-Wallis one-way analysis of variance on ranks]). | | | | | |

### Discussion

In this Example, it has been demonstrated that the targeted double mutant HSV strain, Myb34.5, effectively killed malignant glioma cells both *in vitro* and *in vivo,* while retaining a high degree of safety in terms of neurotoxicity on direct intracranial inoculation in mice. This strain also exhibited minimal replication in primary arrested fibroblasts and marked induction of replication in cycling cells, similar to the induction seen with an HSV characterized by a single RR mutation. In tumor cells, it also replicated vigorously, with improved oncolytic efficacy compared to the parental double RR/γ34.5 mutant, MGH1. This is relevant as MGH1 and other γ34.5 mutants may have limited efficacy due to the lower viral yields obtained from infected cells (Kramm, C.M., *et al*., *supra*). Perhaps most importantly, Myb34.5, like MGH1 or other γ34.5 mutants, demonstrated little pathogenicity in mice at intracerebral doses of 10⁷ PFU, remaining neuroattenuated. Therefore, Myb34.5 exhibits improved oncolytic efficacy compared to the parental mutant MGH1, the marker-rescued revertant MybRevt, and MGH1's parental mutant R3616, while maintaining characteristics of neuroattenuation, with an LD₅₀ of>10⁷ PFU. This value is qualitatively similar to those observed with γ34.5 mutants, although strict quantitative comparisons were limited by the technical inability to accurately determine an LD₅₀ for the latter group of mutants.

A major objective in cancer gene therapy is to identify viral mutants that provide significant anticancer effects while at the same time demonstrating minimal side effects and toxicity towards normal cells and tissues. This feat has been accomplished by engineering replication-defective viral vectors, which should display minimal toxicity toward infected normal and tumor cells, and endowing them with the ability to express anticancer genes to achieve biologic effects (Moriuchi, S., *et al*., *Cancer Res*. *58*:5731-5737 (1998)). When applied as inocula to large human tumor masses, such vectors do not diffuse well due to their size, thus limiting anticancer effects to cells located in proximity to the injection tract (Bobo, R.H., *et al*., *Proc. Natl*. *Acad*. *Sci*. *USA 91*:2076-2080 (1994); Puumalainen, A.M., *et al*., *Hum*. *Gene*. *Ther*. *9*:1769-1774 (1998)).

One potential solution to this problem consists of the use of replication-conditional (oncolytic, replication-restricted) viral mutants that maintain the ability to replicate in a relatively selective fashion in tumor or mitotic cells while being restricted in their ability to replicate in normal cells. Such viral mutants would thus propagate from initially infected tumor cells to surrounding tumor cells, thus achieving a larger volume of distribution and enhanced anticancer effects. However, one might expect increased toxicity with these mutants. In order to minimize the potential toxicities associated with replication-conditional HSV-1, deletion of the endogenous γ34.5 genes has been shown to significantly limit or eliminate the risk of encephalitis or meningitis upon intracerebral injection in rodents (Chou, J., *et al*., *Science 250*:1262-1266 (1990); Markert, J.M., *et al*., *Neurosurgery 32*:597-603 (1993); Markovitz, N.S., *et al*., *J*. *Virol*. *71*:5560-5569 1997)) and *Aotus* monkeys (Mineta, T., *et al*., *Nat*. *Med*. *1*:938-943 (1995)). Further, in a phase I clinical trial in humans afflicted with malignant brain tumors, this type of mutant has not shown evidence of ill effects (Maruza, R.L., *personal communication*).

One concern, however, is that complete elimination of endogenous γ34.5 function would also limit the anticancer effect of HSV. In published experiments, this limitation was shown for rat 9L gliosarcoma cells both *in vitro* and *in vivo* (Kramm, S., *et al*., *Hum*. *Gene Ther*. *8*:2057-2068 (1997)). In the present Example, we also tested killing mediated by HSV mutants with deletions of the γ34.5 function (MGH1 and MybRevt) against a panel of five human glioma cell lines (Table 2). As expected, these mutants were relatively limited in their oncolytic efficacy, compared to the wild-type F strain. Similar findings were also observed with R3616, MGH1's parental strain (Qureshi, N. and Chiocca, E.A., *unpublished data*). However, the oncolytic efficacy of MGH1 was restored to levels that were closer to those observed with the wild-type F strain upon insertion of a single γ34.5 gene under B-*myb* promoter control.

The significance of this result is that inoculation of Myb34.5 into tumors is expected to produce more extensive oncolysis than inoculation of MGH1, R3616, or other γ34.5 mutants into tumors.

In fact, the antitumor efficacy of Myb34.5 *in vivo* was found to be quantitatively similar to that of a single RR mutant (hrR3), suggesting that in tumor cells, active expression of the γ34.5 product returns Myb34.5 to a γ34.5-positive phenotype. However, because hrR3 is a simple insertional mutant, Myb34.5 offers the theoretical advantage of being less prone to recombinatorial repair to wild-type in the presence of latent pre-existing, or subsequent HSV infection. Even in the unlikely event that repair of the ICP6 locus via homologous recombination might occur, the B-*myb*/γ34.5 insert would be excised and return Myb34.5 to the γ34.5 deleted genotype of R3616, the parental virus for MGH1.

Published results have demonstrated that at last one function of γ34.5 is to preclude the host cell's response to viral infection, namely, the triggering of host protein synthesis shutoff in an apoptosis-like response (Chou, J., *et al*., *Proc*. *Natl*. *Acad*. *Sci*. *USA 92*:10516-10520 (1995); Chou, J., *et al*., *Science 250*:1262-1266 (1990); Chou, J. and Roizman, B., *Proc*. *Natl*. *Acad*. *Sci*. *USA 89*:3266-3270 (1992)). A similar function is widespread among pathogenic viruses (Cosentino, G.P., *et al*., *Proc*. *Natl*. *Acad*. *Sci*. *USA 92*:9445-9449 (1995); Gale, M., *el al*., *Mol*. *Cell*. *Biol*. *18*:5208-5218 (1998); Katze, M.G., *Trends Microbiol*. *3*:75-78 (1995); Sharp, T.V., *et al*., *Nucleic Acids Res*. *21*:4483-4490 (1993)). While γ34.5 is nonessential for viral growth in culture in Vero cells, it enables the virus to spread in the mouse central nervous system (Kesari, S., *et al*., *J*. *Gen*. *Virol*. *79*:525-536 (1998); Kesari, S., *et al*., *J*. *Neurosci*. *16*:5644-5653 (1996); Markovitz, N.S., *et al*., *J*. *Virol*. *71*:5560--5569 (1997)) and maps to a region of the HSV genome previously implicated in CNS replication (Centifanto-Fitzgerald, Y.M., *et al*., *J*. *Exp. Med*. *155*:475-489 (1982); Markovitz, N.S., *et al*., *J*. *Virol*. *71*:5560--5569 (1997)). This may be due to the fact that the γ34.5-encoded protein inhibits the double-stranded RNA-dependent kinase. On exposure to double-stranded RNA molecules, as seen commonly with viral infection, RNA-dependent kinase phosphorylates the alpha subunit of elongation initiation factor 2, resulting in inhibition of protein synthesis (Chou, J., *et al*., *Science 250*:1262-1266 (1990); Chou, J., *et al*., *J*. *Virol*. *68*:8304-8311 (1994); Chou, J. and Roizman, B., *Proc*. *Natl*. *Acad Sci*. *USA 89*:3266-3270 (1992)). Infection of cells of neuronal origin with mutants incapable of expressing γ34.5 results in shutoff of cellular protein synthesis, with the resultant limitation of viral production.

One critical aspect of the present study was to show restoration of γ34.5 function by B-*myb* promoter transcriptional control. This was done by demonstrating that infection of cells with MGH1 and MybRevt resulted in suppression of protein synthesis, while protein synthesis was restored when Myb34.5 was the infecting virus. Further evidence for the novel B-*myb* transcriptional dependence of γ34.5 function to the cell cycle was provided by the lovastatin arrest experiments. These clearly showed that under growth arrest conditions, when B-*myb* transcriptional activity is minimal, titers of Myb34.5 were similar to those of MGH1 and MybRevt and dissimilar from those of the wild-type F strain and hrR3. However, when cells were serum stimulated, titers of Myb34.5 increased by 3 orders of magnitude, approaching titers observed with strains F and hrR3, while titers of the γ34.5 mutants (MGH1 and MybRevt) increased only slightly. It was notable that the basal level of replication of Myb34.5 was lower than that of hrR3 in quiescent cells and was more quantitatively similar to that of the RRγ34.5 mutant MGH1. Myb34.5 demonstrated a higherfold induction of replication in cycling cells than hrR3, while MGH1 and MybRevt showed minimal induction, suggesting that the effects of the Myb34.5 construct exceed the effect of simple complementation of ribonucleotide reductase.

These results confirm the hypothesis that the B-*myb* promoter restricts viral replication in quiescent cells and redirects viral replication to cycling cells. In the context of brain tumor therapy, Myb34.5 would thus replicate at relatively low levels (similar to the levels observed with MGH1) in cells that are quiescent, but infection of dividing brain tumor cells would produce a significant increase in viral titers, thus providing a therapeutic advantage over MGH1 or other γ34.5 mutants. Infection of normal brain cells can occur with γ34.5 mutants (Kesari, S., *et al*., *J*. *Gen*. *Virol*. *79*:525-536 (1998); Kesari, S., *et al*., *J*. *Neurosci*. *16*:5644-5653 (1996); Markovitz, N.S., *et al*., *J*. *Virol*. *71*:5560-5569 (1997)), and one would expect Myb34.5 action to mimic that of these mutants in quiescent infected neural cells. In fact, our *in vitro* and *in vivo* studies do show that Myb34.5 replication in cultured neurons and in the brains of mice is similar to that of the γ34.5 mutant viruses (MGH1, MybRevt, and R3616) and dissimilar from that of wild-type F strain and hrR3.

An alternative explanation may be that observed effects of Myb34.5 are due to the replacement of the two endogenous γ34.5 genes by a single γ34.5 gene, and by use of a promoter (B-*myb*) that is weaker than the endogenous HSV γ34.5 promoter, and whose characteristics of strict cell cycle regulation are disrupted in the context of the HSV genome. Although formal exclusion of this possibility would require extensive additional experimentation with mutant B-*myb* promoters, we believe that it remains unlikely in view of the current experimental data. If this explanation were true, then one would predict: (i) Myb34.5 replication in quiescent cells to be higher than that of γ34.5 deletion mutants because of low level expression of the γ34.5 gene by a deregulated B-*myb* promoter in the former mutants; (ii) the inhibition ofhost protein synthesis shutoff observed in neuroblastoma cells infected with Myb34.5 (Fig. 2) to be less pronounced than that observed in cells infect with F or hrR3 because of lower expression of the single γ34.5 gene with a weaker promoter compared to the robust expression achieved by the two γ34.5 genes driven by the endogenous HSV promoter; (iii) the replication of Myb34.5 in U373 cells, known to severely restrict replication of γ34.5 mutants (Mohr, I. and Gluzman, Y., *EMBO J*. *15*:4759-4766 (1996)), to also be somewhat restricted by the weaker expression of the γ34.5 gene in Myb34.5 compared to that of F or hrR3; and (iv) the differential in replication between quiescent and cycling cells to be higher for the hrR3 or F strain (expressing two copies of γ34.5 driven by the endogenous HSV promoter) than that for Myb34.5 (expressing one copy of γ34.5 driven by a deregulated B-*myb* promoter).

The experimental data in this Example does not agree with the aforementioned predictions. The most likely explanation for the observed results is that the B-*myb* promoter remains regulated in a relatively tight fashion even in the context of the HSV genome, thus leading to minimal, if any, expression of γ34.5 in quiescent cells and to levels of expression in cycling and tumor cells that appear functionally similar to those observed with viral strains with intact γ34.5 function.

It has recently been shown that HSV mutants can be engineered to function as vectors. This allows them to express not only viral oncolytic functions, but also additional anticancer effects, thereby increasing their therapeutic efficacy (Chase, M., *et al*., *Nat*. *Biotechnol*. *16*:444-448 (1998)). Clearly, Myb34.5 may also provide a suitable backbone for the addition of anticancer genes, such as those that activate prodrugs. As tumor-selective promoters are identified, it would be relatively easy to use these to control expression of the γ34.5 gene or other virulence genes in order to further restrict viral production to tumor versus normal cells. The approach described here may also be used to restrict virulence to specific cell types in a tissue, by employing cell-specific promoters.

The B-*myb* promoter contains a consensus E2F binding site, is strictly regulated in cycling cells, and is in fact repressed in G₀ (Lam, E.W. and Watson, R.J., *EMBO J*. *12*:2705-2713 (1993); Lam, E.W., *et al*., *Gene 160*:277-281 (1995); Bennett, J.D., *et al*., *Oncogene 13*:1073-1082 (1996)). A replication-defective adenovirus containing an E2F-responsive promoter has been used to demonstrate tumor-specific gene expression, relative not only to quiescent neuronal tissue but also to nontransformed normal cycling cells (Parr, M.J., *et al*., *Nat. Med*. *3*:1145-1149 (1997)). Alteration of some portion of the cell cycle regulatory p 16/retinoblastoma/cdk4 pathway, which regulates E2F, appears to be a near universal event in human gliomas as well as many other tumor types, and provides an excellent substrate for targeting tumor specific expression of viral gene products (He, J., *et al*., *Cancer Res*. *54*:5804-5807 (1994); Ueki, K., *et al*., *Cancer Res*. *56*:150-153 (1996)).

Another HSV-1 mutant in which an albumin promoter was employed to regulate the expression of ICP4 toward hepatocytes has been described (Miyatake, S., *et al*., *J*. *Virol*. *71*:5124-5132 (1997)). The primary difference from the strategy described in the present report is the use of a promoter that may be considered tumor or cell cycle specific instead of hepatocyte specific, and the use of an HSV gene that is directly related to virulence (y34.5) rather than an essential transcription factor, such as ICP4. In contrast to many viral vectors under investigation for treatment of glioblastoma, the exemplified virus, designated Myb34.5, is replication competent, and targeted virulence is obtained by regulating viral replication and direct oncolysis. In this respect, Myb34.5 represents a novel, targeted oncolytic herpesvirus and adds to two recently described tumor-selective, oncolytic adeno- and reoviruses (Bischoff, J.R., *et al*., *Science 274*:373-376 (1996); Coffey, M.C., *et al*., *Science 282*:1332-1334 (1998)). The E1B-defective adenovirus ONYX-015 is thought to depend on alterations of the p53 tumor suppressor pathway for efficient replication in tumor cells,although this mechanism has recently been called into question (Goodrum, F.D., *et al*., *J*. *Virol*. *72*:9479-9490 (1998)).

It has been shown that a reovirus strain selectively replicates in cells with an activated *ras* pathway (Coffey, M.C., *et al*., *supra*). Myb34.5 may take advantage of alterations of the p16/cdk4/RB/E2F pathway, and adds to the possibility that multiple tumor genetic alterations may be targeted by different viral treatment strategies. The strategy of using cell-specific or tumor-specific promoters to drive expression of the γ34.5 gene may also be suitable as a means to eliminate selected cell populations *in vivo.* Finally, the finding of increased safety combined with potent antitumor efficacy suggests that Myb34.5 is an excellent candidate for the treatment of malignant tumors.

Modifications of the above-described modes for carrying out the invention that are obvious to persons of skill in medicine, virology, molecular biology, immunology, pharmacology, and/or related fields are intended to be within the scope of the following claims.

All publications and patents mentioned in this specification are indicative of the level of skill of those skilled in the art to which this invention pertains. All publications and patents are herein incorporated by reference to the same extent as if each individual publication or patent application were specifically and individually indicated to be incorporated by reference.

Although the foregoing invention has been described in some detail by way of illustration and example for purposes of clarity of understanding, it will be obvious that certain changes and modifications can be practiced within the scope of the appended claims.

## Claims

1. A herpes viral mutant comprising:
(a) a deletion or inactivating mutation in both copies of the gene encoding γ34.5; and
(b) an insertion of at least one copy of the γ34.5 gene under the transcriptional control of a cell-specific and/or tumour-specific promoter.

2. The herpes viral mutant of claim 1, wherein said herpes virus is herpes simplex virus type 1.

3. The herpes viral mutant of claim 1, wherein said herpes virus is herpes simplex virus type 2.

4. The herpes viral mutant of claim 1, further comprising at least one additional endogenous deletion or inactivating mutation of a herpes viral gene.

5. The herpes viral mutant of claim 2, further comprising at least one additional endogenous deletion or inactivating mutation of a herpes viral gene.

6. The herpes viral mutant of claim 5, wherein said endogenous deletion or inactivating mutation of a herpes viral gene is a gene that encodes ribonucleotide reductase (RR), thymidine kinase (TK), uracil DNA glycosylase (UNG), or dUTPase.

7. The herpes viral mutant of claim 5 or claim 6, further comprising a transgene whose product is cytotoxic to neoplastic cells.

8. The herpes viral mutant of claim 7, wherein said transgene encodes a product capable of activating or enhancing a chemotherapeutic agent, a cytokine gene, a tumour suppresser gene, or a tumoricidal gene selected from the group consisting of diphtheria toxin, pseudomonas toxin, anti-angiogenesis genes, tumour vaccination genes, radiosensitivity genes, antisense RNA, or ribozymes.

9. The herpes viral mutant of claim 8, wherein said transgene is inserted in the original γ34.5 deletion or anywhere in the herpes UL40 locus.

10. The herpes viral mutant of claim 8, wherein said transgene encodes a suicide gene that activates a chemotherapeutic agent.

11. The herpes viral mutant of claim 10, wherein said suicide gene is mammalian cytochrome P450.

12. The herpes viral mutant of claim 11, wherein said mammalian cytochrome P450 is P450 2B1, P450 2B6, P450 2A6, P450 2C6, P450 2C8, P450 2C9, P450 2C11, or P450 3A4.

13. The herpes viral mutant of claim 2, wherein said tumour-specific promoter is DF3 (MUC1); AFP; CEA PSA; tyrosinase, B-*myb*, or c-*erb*B2.

14. The herpes viral mutant of claim 13, wherein said tumour-specific promoter is B-*myb*.

15. The herpes viral mutant Myb34.5.

16. The herpes viral mutant of claim 2, wherein said cell-specific promoter is vascular endothelial growth factor (VEGF) receptor (flk1) promoter expressed in endothelial cells; insulin promoter expressed in beta cells of the pancreas; gonadotropin-releasing hormone receptor gene expressed in cells of the hypothalamus; matrix metalloproteinase 9 promoter, expressed in osteoclasts and keratinocytes; parathyroid hormone receptor expressed in bone cells; or dopamine beta-hydroxylase promoter expressed in noradrenergic neurons.

17. The use of a viral mutant as claimed in any one of claims 1-6 or 13-15 in the manufacture of a medicament for selectively killing neoplastic cells that overexpresses a known tumour-specific promoter.

18. The use of a viral mutant as claimed in any one of claims 10-12 in the manufacture of a medicament for selectively killing neoplastic cells that over express a known tumour-specific promoter.

19. The method of claim 18, wherein said cytochrome P450 is P450 2B1.

20. The use of claim 18 or claim 19, wherein said chemotherapeutic agent is cyclophosphamide or ifosfamide.

21. The use of a herpes viral mutant, comprising:
(a) a deletion or inactivating mutation in a gene encoding γ34.5; and
(b) an insertion of at least one copy of said γ34.5 gene under the transcriptional control of said cell-specific promoter, such that said promoter drives expression of said γ34.5 gene; in the manufacture of a medicament for selectively eliminating a target cell population that overexpresses a known cell-specific promoter.

22. The use of claim 21, wherein said herpes viral mutant further comprises at least one additional endogenous deletion or inactivating mutation of a herpes viral gene.

23. The use of claim 22, wherein said additional endogenous deletion or inactivating mutation of a herpes viral gene is in a gene that encodes ribonucleotide reductase (RR), thymidine kinase (TK), uracil DNA glycosylase. (UNG), or dUTPase.

24. The use of claim 22, wherein said cell-specific promoter is: vascular endothelial growth factor (VEGF) receptor (flk1) promoter expressed in endothelial cells; insulin promoter expressed in beta cells of the pancreas; gonadotropin-releasing hormone receptor gene expressed in cells of the hypothalamus; matrix metalloproteinase 9 promoter, expressed in osteoclasts and keratinocytes; parathyroid hormone receptor expressed in bone cells; or dopamine beta-hydroxylase promoter expressed in noradrenergic neurons.

## Patentansprüche

1. Herpesvirusmutant, welcher Folgendes aufweist:
(a) eine Deletion oder deaktivierende Mutation in beiden Kopien des Gens, welches γ34.5 codiert; und
(b) eine Insertion von zumindest einer Kopie des γ34.5 Gens unter der Regulation der Transkription durch einen Zell-spezifischen und/oder Tumor-spezifischen Promotor.

2. Herpesvirusmutant nach Anspruch 1, wobei das Herpesvirus das Herpessimplex-Virus Typ 1 ist.

3. Herpesvirusmutant nach Anspruch 1, wobei das Herpesvirus das Herpessimplex-Virus Typ 2 ist.

4. Herpesvirusmutant nach Anspruch 1, welches ferner zumindest eine zusätzliche endogene Deletion oder deaktivierende Mutation eines Herpesvirusgens aufweist.

5. Herpesvirusmutant nach Anspruch 2, welches ferner zumindest eine zusätzliche endogene Deletion oder deaktivierende Mutation eines Herpesvirusgens aufweist.

6. Herpesvirusmutant nach Anspruch 5, wobei die endogene Deletion oder deaktivierende Mutation eines Herpesvirusgens ein Gen ist, welches Ribonukleotid-Reduktase (RR), Thymidinkinase (TK), Uracil-DNA-Glykosylase (UNG) oder dUTPase codiert.

7. Herpesvirusmutant nach Anspruch 5 oder 6, welcher ferner ein Transgen aufweist, dessen Produkt zytotoxisch für neoplastische Zellen ist.

8. Herpesvirusmutant nach Anspruch 7, wobei das Transgen ein Produkt codiert, welches in der Lage ist, ein chemotherapeutisches Agens, ein Zytokin-Gen, ein Tumorsuppressorgen oder ein tumorizides Gen, ausgewählt aus der Gruppe bestehend aus Diphtherietoxin, Pseudomonastoxin, Antiangiogenese-Genen, Tumorvakzinationsgenen, Strahlenempfindlichkeitsgenen, Antisense-RNA oder Ribozymen, zu aktivieren oder zu verstärken.

9. Herpesvirusmutant nach Anspruch 8, wobei das Transgen in die ursprüngliche γ34.5 Deletion oder an irgendeiner anderen Stelle des Herpes-UL40 eingefügt wird.

10. Herpesvirusmutant nach Anspruch 8, wobei das Transgen ein Suizidgen codiert, welches ein chemotherapeutisches Agens aktiviert.

11. Herpesvirusmutant nach Anspruch 10, wobei das Suizidgen das Säugetier-Zytochrom P450 ist.

12. Herpesvirusmutant nach Anspruch 11, wobei das Säugetier-Zytochrom P450 P450 2B1, P450 2B6, P450 2A6, P450 2C6, P450 2C8, P450 2C9, P450 2C11 oder P450 3A4 ist.

13. Herpesvirusmutant nach Anspruch 2, wobei der Tumor-spezifische Promotor DF3 (MUC1); AFP; CEA PSA; Tyrosinase, B-*myb* oder c-*erb*B2 ist.

14. Herpesvirusmutant nach Anspruch 13, wobei der tumorspezifische Promotor B-*myb* ist.

15. Herpesvirusmutant Myb34.5.

16. Herpesvirusmutant nach Anspruch 2, wobei der Zell-spezifische Promotor ein vaskulärer Endothelwachstumsfaktor (VEGF) -Rezeptor (flk1) -Promotor exprimiert auf Endothelzellen; ein Insulinpromotor exprimiert in Betazellen des Pankreas; ein Gonadotropin-freisetzendes Hormonrezeptorgen exprimiert in Zellen des Hypothalamus; ein Matrix-Metalloproteinase-9-Promotor exprimiert in Osteoklasten und Keratinozyten; ein Parathyrevidhormonrezeptor exprimiert in Knochenzellen oder ein Dopamin-Beta-Hydroxylase-Promotor exprimiert in noradrenergischen Neuronen ist.

17. Verwendung eines Virusmutanten nach einem der Ansprüche 1-6 oder 13-15 bei der Herstellung eines Medikamentes für das selektive Abtöten neoplastischer Zellen, welche einen bekannten Tumor-spezifischen Promotor überexprimieren.

18. Verwendung eines Virusmutanten nach einem der Ansprüche 10-12 bei der Herstellung eines Medikamentes für das selektive Abtöten neoplastischer Zellen, welche einen bekannten Tumor-spezifischen Promotor überexprimieren.

19. Verfahren nach Anspruch 18, wobei das Zytochrom P450 P450 2B1 ist.

20. Verwendung nach Anspruch 18 oder 19, wobei das chemotherapeutische Agens Cyclophosphamid oder Ifosfamid ist.

21. Verwendung eines Herpesvirusmutanten, welche aufweist:
(a) eine Deletion oder deaktivierende Mutation in einem Gen, welches γ34.5 codiert; und
(b) eine Insertion von zumindest einer Kopie des γ34.5 Gens unter der Regulation der Transkription durch den Zell-spezifischen Promotor, so dass der Promotor die Expression des γ34.5 Gens steuert; bei der Herstellung eines Medikamentes zum selektiven Entfernen einer Zielzellenpopulation, welche einen bekannten Zell-spezifischen Promotor überexprimiert.

22. Verwendung nach Anspruch 21, wobei der Herpesvirusmutant ferner zumindest eine zusätzliche endogene Deletion oder deaktivierende Mutation eines Herpesvirusgens aufweist.

23. Verwendung nach Anspruch 22, wobei die zusätzliche endogene Deletion oder deaktivierende Mutation eines Herpesvirusgens ein Gen ist, welches Ribonukleotid-Reduktase (RR), Thymidinkinase (TK), Uracil-DNA-Glykosylase (UNG) oder dUTPase codiert.

24. Verwendung nach Anspruch 22, wobei der Zell-spezifische Promotor ist: ein vaskulärer Endothelwachstumsfaktor (VEGF) -Rezeptor (flkl) -Promotor exprimiert in Endothelzellen; ein Insulinpromotor, exprimiert in Betazellen des Pankreas; ein Gonadotropin-freisetzendes Hormonrezeptorgen, exprimiert in Zellen des Hypothalamus; ein Matrix-Metalloproteinase-9-Promotor, exprimiert in Osteoklasten und Keratinozyten; ein Parathyrevidhormonrezeptor, exprimiert in Knochenzellen oder ein Dopamin-Beta-Hydroxylase-Promotor, exprimiert in noradrenergischen Neuronen.

## Revendications

1. Mutant d'herpès virus comprenant :
(a) une délétion ou une mutation inactivante dans les deux copies du gène codant γ34.5; et
(b) une insertion d'au moins une copie du gène γ34.5 sous le contrôle transcriptionnel d'un promoteur spécifique de la tumeur et/ou de la cellule.

2. Mutant d'herpès virus selon la revendication 1, dans lequel ledit herpès virus est le virus de l'herpès simplex de type 1.

3. Mutant d'herpès virus selon la revendication 1, dans lequel ledit herpès virus est le virus de l'herpès simplex de type 2.

4. Mutant d'herpès virus selon la revendication 1, comprenant en outre au moins une délétion ou une mutation inactivante endogène supplémentaire d'un gène de l'herpès virus.

5. Mutant d'herpès virus selon la revendication 2, comprenant en outre au moins une délétion ou une mutation inactivante endogène supplémentaire d'un gène de l'herpès virus.

6. Mutant d'herpès virus selon la revendication 5, dans lequel ladite délétion ou mutation inactivante endogène d'un gène de l'herpès virus est un gène qui code pour la ribonucléotide réductase (RR), la thymidine kinase (TK), l'uracile ADN glycosylase (UNG) ou la dUTPase.

7. Mutant d'herpès virus selon la revendication 5 ou la revendication 6, comprenant en outre un transgène dont le produit est cytotoxique pour les cellules néoplasiques.

8. Mutant d'herpès virus selon la revendication 7, dans lequel ledit transgène code pour un produit capable d'activer ou d'augmenter un agent chimiothérapeutique, un gène de cytokine, un gène suppresseur de tumeur ou un gène tumoricide choisi parmi le groupe constitué de toxine diphtérique, de toxine de pseudomonas, de gènes anti-angiogenèse, de gènes de vaccination de tumeur, de gènes de radiosensibilité, d'ARN antisens ou de ribozymes.

9. Mutant d'herpès virus selon la revendication 8, dans lequel ledit transgène est inséré dans la délétion originale γ34.5 ou n'importe où dans le locus UL40 de l'herpès.

10. Mutant d'herpès virus selon la revendication 8, dans lequel ledit transgène code pour un gène suicide qui active un agent chimiothérapeutique.

11. Mutant d'herpès virus selon la revendication 10, dans lequel le gène suicide est le cytochrome mammalien P450.

12. Mutant d'herpès virus selon la revendication 11, dans lequel ledit cytochrome mammalien P450 est P450 2B1, P450 2B6, P450 2A6, P450 2C6, P450 2C8, P450 2C9, P450 2C11 ou P450 3A4.

13. Mutant d'herpès virus selon la revendication 2, dans lequel ledit promoteur spécifique de tumeur est DF3 (MUC1); AFP; CEA; PSA; tyrosinase, B-*myb* ou c-*erb*B2.

14. Mutant d'herpès virus selon la revendication 13, dans lequel ledit promoteur spécifique est B-*myb*.

15. Mutant d'herpès virus Myb34.5.

16. Mutant d'herpès virus selon la revendication 2, dans lequel ledit promoteur spécifique de cellule est le promoteur (flkl) du récepteur du facteur de croissance endothélial vasculaire (VEGF) exprimé dans les cellules endothéliales; le promoteur de l'insuline exprimé dans les cellules bêta du pancréas; le gène du récepteur de l'hormone gonadolibérine exprimé dans les cellules de l'hypothalamus; le promoteur de la métalloprotéinase matricielle 9, exprimé dans les ostéoclastes et les kératinocytes; le récepteur de l'hormone parathyroïdienne exprimé dans les cellules osseuses; ou le promoteur de la dopamine bêta-hydroxylase exprimé dans les neurones noradrénergiques.

17. Utilisation d'un mutant viral selon l'une quelconque des revendications 1-16 ou 13-15 dans la fabrication d'un médicament pour tuer sélectivement des cellules néoplasiques qui surexprime un promoteur spécifique de tumeur connu.

18. Utilisation d'un mutant viral selon l'une quelconque des revendications 10-12 dans la fabrication d'un médicament pour tuer sélectivement des cellules néoplasiques qui surexprime un promoteur spécifique de tumeur connu.

19. Procédé selon la revendication 18, dans lequel ledit cytochrome P450 est P450 2B1.

20. Utilisation selon la revendication 18 ou la revendication 19, dans laquelle ledit agent chimiothérapeutique est le cyclophosphamide ou l'ifosfamide.

21. Utilisation d'un mutant d'herpès virus comprenant :
(a) une délétion ou une mutation inactivante dans un gène codant γ34.5; et
(b) une insertion d'au moins une copie dudit gène γ34.5 sous le contrôle transcriptionnel dudit promoteur spécifique de cellule, de sorte que ledit promoteur conduit l'expression dudit gène γ34.5; dans la fabrication d'un médicament pour éliminer sélectivement une population cellulaire cible qui surexprime un promoteur spécifique de cellule connu.

22. Utilisation selon la revendication 21, dans laquelle ledit mutant d'herpès virus comprend en outre au moins une délétion ou une mutation inactivante endogène supplémentaire d'un gène d'herpès virus.

23. Utilisation selon la revendication 22, dans laquelle ladite délétion ou mutation inactivante endogène supplémentaire d'un gène d'herpès virus est dans un gène qui code pour la ribonucléotide réductase (RR), la thymidine kinase (TK), l'uracile ADN glycosylase (UNG) ou la dUTPase.

24. Utilisation selon la revendication 22, dans laquelle ledit promoteur spécifique de cellule est : le promoteur (flk1) du récepteur du facteur de croissance endothélial vasculaire (VEGF) exprimé dans les cellules endothéliales; le promoteur de l'insuline exprimé dans les cellules bêta du pancréas; le gène du récepteur de l'hormone gonadolibérine exprimé dans les cellules de l'hypothalamus; le promoteur de la métalloprotéinase matricielle 9, exprimé dans les ostéoclastes et les kératinocytes; le récepteur de l'hormone parathyroïdienne exprimé dans les cellules osseuses; ou le promoteur de la dopamine bêta-hydroxylase exprimé dans les neurones noradrénergiques.
